# FASCICULE DE BREVET EUROPEEN

(11) **EP 4 373 467 B1**
(45) Date de publication et mention de la délivrance du brevet: **08.10.2025**
(21) Numéro de dépôt: 22755080.3
(22) Date de dépôt: 19.07.2022
(51) Int. Cl.: A61K 8/9789, A61K 8/55, A61Q 19/00, A61Q 19/02, A61Q 19/08

(54) **EXTRAIT DE FEUILLES DE THE NOIR, COMPOSITIONS LE COMPRENANT ET SES UTILISATIONS COSMETIQUES**
EXTRAKT AUS BLÄTTERN AUS SCHWARZEM TEE, ZUSAMMENSETZUNGEN DAMIT UND KOSMETISCHE VERWENDUNGEN DAVON
BLACK TEA LEAF EXTRACT, COMPOSITIONS COMPRISING SAME AND COSMETIC USES THEREOF

(30) Priorité: 22.07.2021 FR 2107927
(43) Date de publication de la demande: 29.05.2024
(73) Titulaire: ISP Investments LLC, Wilmington, Delaware 19805 (US)
(72) Inventeur: MOURET, Laura, 06410 BIOT (FR); OGER, Elodie, 06600 ANTIBES (FR)
(74) Mandataire: Miquel, Corinne
(86) Numéro de dépôt international: PCT/EP2022/070181
(87) Numéro de publication internationale: WO 2023/001812

(56) Documents cités:
- WO-A2-2013/092117
- JP-A- 2006 191 924
- US-A1- 2020 129 579

## Description

### Domaine technique

La présente demande concerne le domaine de la cosmétique et plus particulièrement des agents actifs d'origine naturelle entrant dans la préparation de formulations cosmétiques pour améliorer l'aspect de la peau ou la protéger. La présente demande se rapporte à un extrait de thé noir (*Camellia sinensis*) enrichi en petits ARN, en sucres, en composés phénoliques, en acides organiques, en acides aminés, et en théanine et à un procédé de préparation d'un tel extrait. La présente demande porte également sur des compositions cosmétiques comprenant un tel extrait. La présente demande porte encore sur des utilisations cosmétiques de compositions comprenant un extrait de thé noir de toute provenance, à l'exclusion du thé noir provenant de l'Ile Maurice, pour le soin de la peau, du cuir chevelu et des phanères et plus particulièrement pour protéger la peau des agressions externes et de l'oxydation, lutter contre les signes du vieillissement cutané, augmenter la photoprotection, éclaircir la peau et améliorer l'hydratation de la peau.

### Arrière-plan technique de l'invention

La plante de l'espèce *Camellia sinensis,* communément appelée plante à thés, comprend trois variétés botaniques : Camellia sinensis var. assamica, Camellia sinensis var. sinensis (Yunnan) et Camellia sinensis var. Cambodiensis.

Les feuilles du théier (*Camellia sinensis*) sont utilisées pour préparer différentes sortes de thés. Ainsi, c'est le traitement appliqué aux feuilles récoltées qui va déterminer le type de thé obtenu. Le thé vert n'est pas ou très peu oxydé, le thé blanc légèrement oxydé, le thé oolong est modérément oxydé (entre 10 % et 75 %), le thé noir a subi une oxydation complète.

L'environnement dans lequel pousse la plante a un impact sur son métabolisme et influence la synthèse de phytomolécules. L'adaptation de la plante à son milieu est le fruit d'une régulation épigénétique fine, qui passe, en outre, par l'expression de petits ARN capables de réguler rapidement l'expression des gènes de la plante et ainsi permettre l'adaptation au stress et à l'environnement.

Ainsi, la saison, le lieu de culture et le type de feuilles récoltées influence la composition chimique du thé noir. Par exemple, ce sont les bourgeons et les plus jeunes feuilles du thé qui ont la teneur la plus élevée en polyphénols. Ces dernières contiennent environ 15 % en poids sec de catéchines, dont 10 % de EGCG (Liang Zhang, Comprehensive Reviews in Food Science and Food Safety, Vol.18, 2019), alors que cette teneur est plus faible dans les feuilles inférieures, plus âgées.

En moyenne, les feuilles de thé fraîches de *Camelia sinensis* provenant de l'Assam en Inde contiennent, entre autres, 22,2 % de polyphénols totaux dont jusqu'à 15 % de catéchines, 17,2 % de protéines, 4,3 % de théine, 27 % de fibres, 0,5 % d'amidon et 3,5 % de sucres réducteurs (Duke, J .A., Handbook of Energy Crops, 1983, Purdue University, Unpublished https://hort.purdue.edu/newcrop/duke_energy/Camellia_sinensis.html).

Le thé noir est connu pour ses bienfaits sur la santé, eux-mêmes attribués à sa forte concentration en molécules antioxydantes et autres composés ayant la propriété de réduire l'inflammation et le risque d'apparition de maladies chroniques.

Dans le thé noir, on trouve en moyenne, environ 10,2 mg/g de catéchines, 28,54 mg/g de théine, 1 mg/g de théobromine, 10,70 mg/g de théflavines, 0,65 mg/g de théanine, mais les quantités peuvent varier considérablement (Liang Zhang, Comprehensive Reviews in Food Science and Food Safety, Vol.18, 2019).

On définit le thé noir dans la présente demande comme un thé provenant de feuilles de l'espèce *Camellia sinensis* qui ont subi une oxydation complète. Le thé noir peut être préparé selon des méthodes artisanales ou mécanisées qui ont en commun une étape de pré-séchage ou de flétrissement puis un déchiquetage ou roulage des feuilles ayant pour but de casser les cellules et libérer les enzymes responsables de l'oxydation et en particulier la polyphénol oxydase. L'oxydation du thé noir a lieu lorsque les polyphénols contenus dans les vacuoles cellulaires rentrent en contact avec des enzymes de dégradations. La réaction chimique qui se produit alors, transforme les catéchines du thé principalement en théaflavines et en théarubigine. La chlorophylle est également transformée en phéophytine qui pigmente le thé en noir. L'oxydation a lieu dans une atmosphère chaude et très humide. L'oxydation est ensuite stoppée par chauffage des feuilles à 90°C.

De nombreux extraits de feuilles de thé sont décrits dans la littérature en particulier pour leur richesse en composés phénoliques incluant les catéchines. La plupart des méthodes d'extraction de l'art antérieur utilisent des solvants organiques pour leurs capacités à extraire les polyphénols de type catéchine (WO2006/111666, KR2016021734A, KR2018125828A, JP2006191924A). Les extraits ainsi obtenus sont riches en polyphénols et flavonoïdes, mais ne contiennent pas ou peu de molécules phénoliques de type acides phénoliques, ou encore de molécules organiques telles que des sucres, des acides aminés ou encore des oligo-ribonucléotides. En effet, les molécules telles que les sucres, les acides aminés ou les oligo-ribonucléotides sont mieux extraites par les solvants polaires et idéalement par les solutions aqueuses. Ainsi toutes ces phytomolécules, qui sont reconnues pour leurs effets bénéfiques sur la peau ne sont pas efficacement extraites par les méthodes d'extraction des feuilles de théier décrites dans l'art antérieur.

La demande des utilisateurs de disposer de produits cosmétiques aussi naturels que possible mais présentant une efficacité équivalente, voire meilleure que les produits synthétiques, augmente rapidement. Dans la présente demande les extraits décrits sont 100 % naturels afin de répondre aux exigences des consommateurs.

Un problème que se propose de résoudre l'invention est de fournir un nouvel extrait aqueux de thé noir répondant à l'exigence du marché de la cosmétique actuel concernant les critères de naturalité et présentant néanmoins une efficacité biologique remarquable, ainsi qu'une absence de toxicité.

En effet, une trop forte concentration en composés phénoliques tels que les catéchines peut provoquer des phénomènes de phototoxicité ou limiter la stabilité de l'extrait dans le temps. Par exemple, la principale forme de catéchine présente dans le thé vert, l'épigallocatéchine Gallate (EGCG) est ainsi hautement instable en solution aqueuse. Sous l'effet de différents facteurs comme l'oxygène, la lumière du soleil ou les changements de température ou de pH, elle se dégrade et donne naissance à des superoxydes et des produits oxydés. Cela conduit à la formation de dimères responsables du brunissement des solutions (J. Zeng et al. Molecules 2018, 23, 2394) .

Les extraits décrits dans la présente demande ont une composition originale en composés phénoliques par rapport aux extraits décrits dans l'art antérieur. La concentration en composés phénoliques est notablement plus faible, majoritairement composée d'acides phénoliques et comprenant très peu du polyphénol majoritaire du thé ; à savoir les catéchines. De plus, les extraits décrits dans la présente demande présentent également une bonne stabilité dans le temps et une absence de toxicité.

Un autre problème que se propose de résoudre l'invention est de fournir un nouvel extrait aqueux de thé noir enrichi en composés connus pour leur efficacité sur la peau comme les sucres, les acides organiques, les acides aminés, la théine et la théanine ainsi que des petits ARN.

Les inventeurs ont développé un nouvel extrait de thé noir spécifiquement enrichi en petits ARN, en sucres, en composés phénoliques et organiques, en acides organiques, en acides aminés, en théanine, obtenu par un procédé ne présentant pas les inconvénients des procédés de l'art antérieur, comme par exemple l'utilisation de détergents et de solvants potentiellement toxiques pour un usage en cosmétique.

L'extrait ainsi obtenu peut être utilisé en cosmétique pour le soin de la peau, du cuir chevelu et des phanères, pour obtenir de multiples bénéfices et plus particulièrement pour protéger la peau des agressions externes et de l'oxydation, lutter contre les signes du vieillissement cutané, augmenter la photoprotection, éclaircir la peau, améliorer l'hydratation de la peau, renforcer la fonction barrière ou encore apaiser la peau.

### Résumé de l'invention

L'invention a pour premier objet un extrait de feuilles de thé noir de l'espèce *Camellia sinensis* comprenant de 10 à 350 mg/kg de petits ARN d'une longueur d'au maximum 150 nucléotides, comprenant au maximum 150 mg/kg de catéchines, de préférence au maximum 120 mg/kg de catéchines et encore plus préférentiellement au maximum 30 mg/kg de catéchines.

L'extrait ci-dessus est susceptible d'être obtenu par le procédé d'obtention suivant, qui est le deuxième objet de l'invention et comprend les étapes suivantes :
a. on met en présence les feuilles de thé noir de l'espèce *Camelia sinensis* préalablement broyées avec de l'eau ;
b. on ajoute de l'acide phytique ;
c. on ajuste le pH à une valeur comprise entre 10 et 11 ;
d. on maintient le mélange sous agitation pendant au moins une heure à une température comprise entre 40 et 80°C ;
e. on purifie le mélange obtenu en c) pour éliminer la matière végétale solide résiduelle et récolter le filtrat ;
f. on ajuste le pH à une valeur comprise entre 6 et 8 ;
g. on traite par du charbon actif en poudre ;
h. on filtre pour éliminer le charbon actif et récolter le filtrat ;
i. on traite le filtrat à l'aide de polyvinylpolypyrrolidones (PVPP) ;
j. on filtre pour éliminer les PVPP et récolter le filtrat ;
k. on contrôle et réajuste le pH du filtrat, si nécessaire, à une valeur comprise entre 6 et 6,5.
l. Optionnellement l'extrait peut être dilué dans un solvant physiologiquement acceptable puis son pH est ajusté à une valeur comprise entre 5,8 et 6,7 préférentiellement entre 6,0 et 6,5, pour obtenir un extrait dilué ayant un poids sec compris entre 4 à 20 g/kg.

L'invention a pour troisième objet une composition comprenant, en tant qu'agent actif, une quantité efficace de l'extrait aqueux dilué de feuilles de thé noir décrit dans la présente demande, et un milieu physiologiquement acceptable.

L'invention a pour quatrième objet l'utilisation non-thérapeutique, cosmétique d'une compositior comprenant, en tant qu'agent actif, une quantité efficace de l'extrait aqueux dilué de feuilles de thé noir décrit dans la présente demande, à l'exclusion du thé noir provenant de l'Ile Maurice, et un milieu physiologiquement acceptable pour le soin de la peau, du cuir chevelu et des phanères et plus particulièrement pour protéger la peau des agressions externes et de l'oxydation, lutter contre les signes du vieillissement cutané, augmenter la photoprotection, éclaircir la peau, ou encore améliorer l'hydratation de la peau.

### Brève description des dessins

L'invention et les avantages qui en découlent seront mieux compris à la lecture de la description et des modes de réalisation non limitatifs qui suivent, illustrés au regard de la figure annexée dans laquelle :

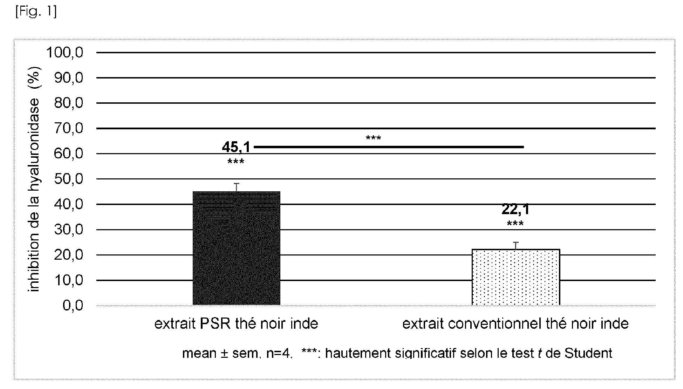

[Fig. 1] Evaluation de l'activité inhibitrice de la hyaluronidase des différents extraits de thé en provenance d'Inde obtenus selon les exemples 1 et 2 par mesure de la turbidité.

### Description détaillée de l'invention

Les inventeurs ont développé un nouvel extrait de thé noir (*Camellia sinensis*) enrichi en petits ARN, en sucres, en composés phénoliques et organiques, en acides organiques, en acides aminés, en théanine, obtenu par un procédé ne présentant pas les inconvénients des procédés de l'art antérieur, comme par exemple l'utilisation de détergents et de solvants potentiellement toxiques en cosmétique.

Les inventeurs ont en outre développé un procédé permettant d'enrichir l'extrait en petits ARN, qui sont des molécules connues pour intervenir dans les régulations épigénétiques et décrites pour leurs activités bénéfiques sur la peau. L'extrait est dépourvu d'ADN qui n'est pas connu pour ses propriétés biologiques bénéfiques sur la peau et peut induire une certaine instabilité de l'extrait.

### Définitions

Tous les termes utilisés dans la présente demande ont le sens le plus largement connu ou le sens indiqué ci-dessous :

On entend par « thé noir » les feuilles de l'espèce *Camellia sinensis entières,* sèches, ayant subies une oxydation complète et de toute provenance géographique.

« Thé noir provenant de l'île Maurice » ou « thé noir d'origine Ile Maurice » désigne un thé cultivé, récolté et transformé dans des plantations situées sur l'Ile Maurice.

« Thé provenant d'Inde » ou « thé noir origine Inde » désigne un thé cultivé, récolté et transformé dans des plantations de thé situés en Inde.

On entend par « extrait de thé noir » ou « extrait de feuilles de thé noir » un extrait aqueux de feuilles de thé noir de l'espèce *Camellia sinensis* comprenant de 10 à 350 mg/kg de petits ARN d'une longueur d'au maximum 150 nucléotides, dépourvu d'ADN, comprenant au maximum 150 mg/kg de catéchines, de préférence au maximum 120 mg/kg de catéchines et encore plus préférentiellement au maximum 30 mg/kg de catéchines.

On entend par « extrait brut de thé noir » que l'extrait obtenu par le procédé décrit dans la présente demande n'a pas été dilué.

Par « petits ARN » ou « ARN de petits poids moléculaires », ou « petits ARN d'une longueur d'au maximum 150 nucléotides » on entend des ARN (acides ribonucléiques) non codants, de petit poids moléculaires, d'une longueur d'au maximum 150 nucléotides, tels que tous types de petits ARN non messagers, simples et/ou doubles brins, par exemple les micro-ARN, les ARN interférents, les introns, les petits ARN nucléaires ou encore tout fragment d'ARN provenant de la plante et extraits par le procédé décrit dans la présente demande. Ainsi, les fragments d'ARN purifiés, de séquences connues ou issus de synthèse ne rentrent pas dans cette définition.

Par « acides organiques » on entend les acides α-hydroxylés (ou AHA), c'est-à-dire des acides carboxyliques dérivés des sucres contenus dans les feuilles de thé, tels que les acides lactique, malique, tartrique, succinique et uronique.

Par « composés phénoliques », on entend les molécules d'origine végétale qui possèdent au moins un cycle aromatique de type phénolique portant un ou plusieurs groupements hydroxyles, tels que les acides phénoliques, les polyphénols et les flavonoïdes tels que les catéchines. Les composés phénoliques sont reconnus pour être de puissantes molécules antioxydantes, aussi bien dans la plante que pour une utilisation cosmétique. Ces métabolites secondaires de la plante sont également produits par les plantes comme mécanismes défensifs lors de stress biotique ou abiotique.

Par « dépourvu d'ADN » on entend que l'extrait de thé noir ne contient pas d'ADN. Ceci a été démontré par un test à la DNAse, enzyme qui dégrade spécifiquement l'ADN et non l'ARN. Le profil électrophorétique après action de la DNase n'est pas modifié, ce qui démontre que l'acide nucléique présent dans l'extrait n'est pas sensible à la DNase et n'est donc pas de l'ADN.

Par « sucres » on entend tous les types de sucres présents dans les plantes, tels que les monosaccharides comme le glucose ou le fructose mais aussi les oligo et polysaccharides. En règle générale, les polysaccharides contiennent plus de dix unités monosaccharidiques, tandis que les oligosaccharides contiennent de trois à dix unités monosaccharidiques.

Par « phytomolécules d'intérêt », on entend l'ensemble des molécules présentes dans les extraits de thé de l'invention et notamment, les petits ARN d'une longueur d'au maximum 150 nucléotides, les sucres, les composés phénoliques, les acides organiques, les acides aminés, la théine et la théanine.

Par catéchines, on entend les polyphénols de type flavonoïdes qui appartiennent à la famille des catéchines, telles que la catéchine (C) mais aussi l'épicatéchine (EC), l'epigallocatéchine (EGC), la gallocatéchine (GC), la catéchine gallate (CG), la gallocatéchine gallate (GCG), l'épigallocatéchine gallate (EGCG), l'épicatéchine gallate (ECG).

Les valeurs numériques en pourcentage sont des pourcentages en poids, c'est-à-dire le poids d'un composé par rapport au poids total du mélange envisagé, sauf spécifié autrement.

Les compositions décrites dans la présente demande peuvent « comprendre », « consister en » ou « consister essentiellement en » les composés essentiels ou les ingrédients optionnels.

« Consister essentiellement en » signifie que la composition ou le composant peut inclure des ingrédients additionnels, mais seulement si les ingrédients additionnels ne modifient pas les caractéristiques de bases ou les nouvelles caractéristiques de la composition ou de l'utilisation décrite dans la présente demande.

Par « application topique » on désigne le fait d'appliquer ou d'étaler l'extrait selon l'invention, ou une composition le contenant, à la surface de la peau ou d'une muqueuse.

« Peau » désigne la peau saine du visage, notamment le contour des yeux et la bouche, le nez, le front, le cou, les mains, mais aussi la peau de l'ensemble du corps.

« Cuir chevelu » désigne la peau saine recouvrant le crâne, incluant les follicules pileux et les espaces cutanés inter-folliculaires.

« Phanères » désigne les produits des follicules pileux (cheveux, cils et poils) ainsi que les ongles, c'est-à-dire les annexes cutanées riches en kératine.

« Eclaircir la peau » signifie diminuer l'intensité de la couleur de la peau liée au contenu en mélanine de l'épiderme, soit de manière homogène, soit de manière localisée en agissant sur des désordres pigmentaires, tels que les tâches de senescence ou lentigos séniles.

Par « quantité efficace » on désigne la quantité minimum d'extrait selon l'invention qui est nécessaire pour obtenir au moins une des activités biologiques recherchées ou pour protéger la peau des agressions externes et de l'oxydation, lutter contre les signes du vieillissement cutané, augmenter la photoprotection, éclaircir la peau et améliorer l'hydratation de la peau, sans que cette quantité soit toxique.

Par « hydratation de la peau », on entend le contenu et la répartition en eau des couches supérieures de l'épiderme.

On entend par « amélioration de l'hydratation cutanée », toutes améliorations des modifications de l'aspect extérieur de la peau dues à la déshydratation comme, par exemple, la sécheresse, les tiraillements et l'inconfort, que cet état soit lié à des facteurs internes ou externes, tels que des conditions environnementales défavorables.

Par « signes du vieillissement cutané » on entend toutes modifications de l'aspect extérieur de la peau dues au vieillissement comme, par exemple, les rides et ridules, les crevasses, les poches sous les yeux, les cernes, le flétrissement, la perte d'élasticité, de fermeté et/ou de tonus de la peau, mais également toutes modifications internes de la peau qui ne se traduisent pas systématiquement par un aspect extérieur modifié comme, par exemple, l'amincissement de la peau, ou toutes dégradations internes de la peau consécutives à des stress environnementaux tels que la pollution et les rayonnements solaires incluant les UV.

Par « signes du vieillissement cutané » on entend également les désordres pigmentaires tels que le lentigo sénile ou lentigo solaire.

Par « agressions externes » on entend les rayonnements solaires, incluant la lumière visible, les rayonnements UV et infrarouges, la pollution, pouvant provenir de l'atmosphère ambiante à l'extérieur ou à l'intérieur des habitations et incluant des particules de différentes tailles (10 µm pour les PM10, 2,5 µm pour les PM 2,5, ou inférieur à 100 nm pour les particules ultrafines) ainsi que plusieurs éléments chimiques (composés organiques volatils, hydrocarbures aromatiques polycycliques, métaux lourds, etc.).

On entend par « améliorer l'aspect de la peau » que le grain de la peau apparaît plus fin, la luminosité plus intense et le teint plus homogène.

Par « physiologiquement acceptable » on entend un milieu composé d'au moins un excipient, un solvant ou un mélange de solvant, adapté pour une mise en contact avec les couches externes de la peau ou des muqueuses, sans toxicité, irritation, réponse allergique indue et similaire ou réaction d'intolérance, et proportionné à un rapport avantage/risque raisonnable.

Il est bien entendu que l'invention concerne les mammifères et plus particulièrement l'être humain.

### Extrait de thé noir

L'invention a pour premier objet un extrait de thé noir de l'espèce *Camellia sinensis* comprenant de 10 à 350 mg/kg de petits ARN d'une longueur d'au maximum 150 nucléotides, au maximum 150 mg/kg de catéchines, de préférence au maximum 120 mg/kg de catéchines et encore plus préférentiellement au maximum 30 mg/kg de catéchines.

Avantageusement, l'extrait de thé noir, en particulier provenant d'Inde, contient des traces de catéchines c'est-à-dire au maximum 0,001% de catéchines par rapport au poids sec de l'extrait, voire encore plus avantageusement ne contient pas de catéchines.

Avantageusement, l'extrait de thé noir comprend de 0,030 à 5 g/kg de composés phénoliques.

L'extrait de thé noir a préférentiellement un poids sec de 4 à 30 g/kg et comprend de 0,2 à 10 g/kg de sucres, de 0,010 à 3 g/kg d'acides aminés totaux, de 0,010 à 2 g/kg de théine et de 0,010 à 2 g/kg de théanine. Plus préférentiellement encore, l'extrait de thé noir est de type aqueux, de couleur ambre à ambre foncé, a un poids sec de 4 à 30 g/kg et comprend de 0,2 à 10 g/kg de sucres, de 0,030 à 2 g/kg d'acides organiques, de 0,010 à 3 g/kg d'acides aminés totaux, de 0,030 à 5 g/kg de composés phénoliques dont au maximum 150 mg/kg de catéchines, de 0,010 à 2 g/kg de théine et 0,010 à 2 g/kg de théanine et de 10 à 350 mg/kg de petits ARN d'une longueur d'au maximum 150 nucléotides, en poids du poids total de l'extrait.

De plus l'extrait de thé noir est dépourvu d'ADN (acide désoxyribonucléique).

Dans un mode de réalisation particulier, l'extrait de thé noir provient de l'Ile Maurice et a un poids sec de 5 à 30 g/kg, comprend de 0,5 à 10 g/kg de sucres totaux, de 0,050 à 2 g/kg d'acides organiques totaux, de 0,050 à 2 g/kg d'acides aminés, de 0,100 à 5 g/kg de composés phénoliques totaux dont au maximum 50 mg/kg de catéchines, de 0,010 à 2 g/kg de théine et 0,010 à 2 g/kg de théanine et de 50 à 350 mg/kg d'ARN de petits poids moléculaires d'une longueur d'au maximum 150 nucléotides.

Dans un mode de réalisation avantageux, l'extrait de thé noir est dilué dans un solvant physiologiquement acceptable pour un usage cosmétique. La dilution est réalisée de façon à ajuster le poids sec de l'extrait à une valeur comprise entre 4 et 20 g/kg.

Le solvant physiologiquement acceptable, est choisi parmi le glycérol, l'éthanol, le propanediol, le butylène glycol, ainsi que leur version naturelle, le dipropylène glycol, les diglycols éthoxylés ou propoxylés, les polyols cycliques ou tout mélange de ces solvants.

De manière avantageuse, le solvant physiologiquement acceptable est choisi parmi le butylène glycol, le propanediol ou la glycérine, obtenus à partir de plantes (version naturelle).

De préférence, l'extrait de thé noir est dilué dans 30 à 50 % de butylène glycol, ou 30 à 50 % de propanediol ou encore 30 à 70 % de glycérine. Lorsque le solvant est la glycérine, de préférence l'extrait de thé noir est dilué dans 70 % de glycérine.

Préférentiellement, l'extrait de thé noir est dilué dans du propanediol obtenu à partir de plante de telle sorte que l'extrait dilué comprenne une concentration finale en propanediol de 30 % ou de 50 %.

Préférentiellement, l'extrait de feuilles de thé noir a été dilué dans un solvant physiologiquement acceptable, a un poids sec compris entre 4 et 20 g/kg et comprend en poids du poids total de l'extrait dilué, de 10 à 250 mg/kg d'ARN de petit poids moléculaire d'une longueur d'au maximum 150 nucléotides, de 0,2 à 5 g/kg de sucres, de 0,010 à 2 g/kg d'acides aminés, de 0,030 à 3 g/kg de composés phénoliques dont au maximum 120 mg/kg de catéchines, de 0,010 à 1 g/kg de théine, de 0,010 à 1 g/kg de théanine.

Plus préférentiellement, l'extrait de feuilles de thé noir ainsi dilué a un poids sec compris entre 4 et 20 g/kg et comprend en poids du poids total de l'extrait dilué, de 10 à 250 mg/kg d'ARN de petit poids moléculaire d'une longueur d'au maximum 150 nucléotides, de 0,2 à 5 g/kg de sucres, de 0,030 à 1 g/kg d'acides organiques, de 0,010 à 2 g/kg d'acides aminés, de 0,030 à 3 g/kg de composés phénoliques dont au maximum 120 mg/kg de catéchines, de 0,010 à 1 g/kg de théine, de 0,010 à 1 g/kg de théanine et est dépourvu d'ADN.

Lorsqu'il provient de l'Ile Maurice et que l'extrait dilué comprend une concentration finale en propanediol de 30 %, l'extrait de thé noir ainsi dilué a un poids sec compris entre 6 à 20 g/kg, et comprend de 50 à 250 mg/kg d'ARN de petit poids moléculaire d'une longueur d'au maximum 150 nucléotides, de 0,8 à 5 g/kg de sucres, 0,2 à 1 g/kg d'acides organiques, de 0,2 à 1 g/kg d'acides aminés, et de 0,5 à 3 g/kg de composés phénoliques, dont au maximum 30 mg/kg de catéchines, de 0, 10 à 100 mg/Kg de théine, de 0,010 à 300 mg/Kg de théanine et est dépourvu d'ADN.

De préférence l'extrait de thé noir provenant de l' Ile Maurice ne contient pas de catéchines.

Lorsqu'il provient d'Inde et que l'extrait dilué comprend une concentration finale en propanediol de 30 %, l'extrait de thé noir ainsi dilué a un poids sec compris entre 4 à 12 g/kg, et comprend de 10 à 100 mg/kg d'ARN de petit poids moléculaire d'une longueur d'au maximum 150 nucléotides, de 0,2 à 3 g/kg de sucres, 0,030 à 0,5 g/kg d'acides organiques, de 0,030 à 0,3 g/kg d'acides aminés, et de 0,050 à 2 g/kg de composés phénoliques, dont au maximum 30 mg/kg de catéchines, de 0,050 à 500 mg/Kg de théine, de 0,010 à 300 mg/Kg de théanine et est dépourvu d'ADN.

L'extrait de thé noir décrit dans la présente demande comprend ainsi une large gamme de phytomolécules présentant des effets bénéfiques sur la peau, sans présenter de risque d'irritation cutanée ou autre dommage pour la santé.

Par exemple, les sucres participent activement à l'hydratation des couches supérieures de l'épiderme, et ce faisant à la résistance aux agressions extérieures, sans présenter aucun effet indésirable. Les extraits de thé de l'invention contiennent plus particulièrement des mono- et des polysaccharides.

*Camellia sinensis* fait partie de la famille des Théaceae qui produit de la théine, un puissant insecticide naturel. La théine est aussi appelée caféine ou 1,3,7-triméthylxanthine ou méthylthéobromine. Au-delà de ses effets reconnus sur le système nerveux et cardio-vasculaire, la théine a également des effets bénéfiques sur la peau en particulier sur le relâchement cutané.

La plante produit également de la théanine, un acide aminé non protéique qui représente 50 % de la quantité totale d'acides aminés présents dans le thé.

L'extrait de thé noir décrit dans la présente demande contient également des composés phénoliques, tels que les polyphénols, les acides phénoliques, les flavonoïdes comme les catéchines, cependant cette famille de molécules a été partiellement ôtée par des étapes de traitement par du charbon et du PVPP. Cet appauvrissement relatif en composés phénoliques permet d'augmenter la stabilité du produit dans le temps et d'obtenir un extrait non phototoxique.

L'extrait de thé noir contient également des acides organiques. Les acides organiques sont des molécules impliquées dans le cycle de Krebs, un processus métabolique crucial dans la respiration cellulaire. Certains acides organiques comme les acides malique, lactique sont appelés acides alpha-hydroxylés (AHA) très connus pour leur effet anti-âge sur la peau, ils améliorent l'apparence et la texture de la peau.

Dans un mode de réalisation particulier l'extrait de thé noir dilué contient au maximum 1,5 g/kg de composés phénoliques dont au maximum 120 mg/kg de catéchines.

Dans un autre mode de réalisation particulier l'extrait de thé noir dilué contient au maximum 1,2 g/kg de composés phénoliques dont au maximum 30 mg/kg de catéchines.

Dans un mode de réalisation particulier l'extrait de thé noir dilué contient au maximum 3 g/kg de composés phénoliques et contient des traces de catéchines au maximum de 0,001% de catéchines par rapport au poids sec de l'extrait de thé noir provenant d'Inde, voire ne contient pas de catéchines pour l'extrait de thé noir provenant d'Inde ou de l'Ile Maurice.

Dans un autre mode de réalisation particulier l'extrait de thé noir dilué contient au maximum 100 mg/kg de composés phénoliques et contient des traces de catéchines au maximum de 0,001% de catéchines par rapport au poids sec de l'extrait de thé noir provenant d'Inde, voire ne contient pas de catéchines pour l'extrait de thé noir provenant d'Inde ou de l'Ile Maurice.

Dans encore un autre mode de réalisation l'extrait de thé noir dilué contient au maximum 50 mg/kg de composés phénoliques et contient des traces de catéchines au maximum de 0,001% de catéchines par rapport au poids sec de l'extrait de thé noir provenant d'Inde, voire ne contient pas de catéchines pour l'extrait de thé noir provenant d'Inde ou de l'Ile Maurice.

Tous les extraits de thé noir décrits dans cette partie de la demande sont susceptibles d'être obtenus par le procédé d'extraction décrit ci-dessous.

### Procédé d'extraction

Les protocoles d'extraction des acides ribonucléiques (ARN) classiquement décrits utilisent des solvants inadaptés à un usage cosmétique (par exemple, Brown, R.A.M., Epis, M.R., Horsham, J.L. et al. Total RNA extraction from tissues for microRNA and target gene expression analysis: not all kits are created equal. BMC Biotechnol 18, 16 (2018). Ces méthodes visent à obtenir des acides nucléiques (ARN ou ADN) complètement purifiés, c'est-à-dire exempts de toute autre molécule d'intérêt tels que les métabolites secondaires, vitamines, sucres ou encore peptides, qui présentent un intérêt cosmétique.

On connait également le document FR2831168 qui décrit un procédé d'obtention d'un extrait de plante riche en acides nucléiques (ADN et ARN). Le procédé utilise des enzymes cellulolytiques.

On connait encore de l'art antérieur les documents de brevet EP1723958 et WO03101376 qui décrivent une composition pour application topique comprenant un oligonucléotide d'ARN double brins de synthèse d'une longueur de 12 à 40 nucléotides, de séquence connue ayant une fonction de siARN (« short interfering »).

On connait encore le document FR 1502361 (également publié sous le numéro WO2017084958) qui décrit un procédé d'obtention d'un extrait aqueux de plantes, enrichi en acides ribonucléiques (ARN) de petit poids moléculaire, pour préparer des compositions cosmétiques. Le procédé utilise de l'EDTA à une concentration comprise entre 2 et 15 mM.

Le procédé décrit dans la présente demande a un faible impact environnemental en ce qu'il utilise comme solvant d'extraction une solution aqueuse et de l'acide phytique qui est une molécule d'origine naturelle.

Le procédé décrit ici permet ainsi d'obtenir un extrait de thé noir d'origine 100 % naturelle tout en maintenant une bonne efficacité d'extraction des phytomolécules contenues dans la plante.

L'extrait de thé noir décrit dans la première partie de cette description est avantageusement obtenu par le procédé d'extraction décrit ci-après.

Les feuilles de thé noir utilisées dans le présent procédé sont sèches, entières ou réduites en poudre.

Lorsqu'elles sont entières, elles sont préalablement broyées, pour être réduites en poudre.

Dans une première étape a) du procédé, on mélange les feuilles de thé noir réduites en poudre avec de l'eau. L'eau utilisée est une eau distillée, déminéralisée ou encore une eau riche en sels minéraux et/ou oligo-éléments. L'eau préférentiellement utilisée est de l'eau distillée.

A l'étape a), le rapport matière végétale / eau est avantageusement compris entre 1 % et 20 %, préférentiellement entre 2 et 10 % et encore plus préférentiellement entre 2 et 5 %.

Le mélange est avantageusement mis sous agitation.

Dans une étape b) on ajoute l'acide phytique au mélange thé-eau de l'étape a).

L'acide phytique est un agent chélatant d'origine naturellement présent dans l'enveloppe des graines, comme les céréales et les légumineuses. Il entraîne la fragilisation et la destruction des membranes pecto-cellulosiques des cellules végétales en séquestrant par complexation les ions divalents tels que les ions calcium qui forment des ponts ioniques entre les molécules de pectines entourant les microfibrilles de cellulose. Ceci a pour conséquence de favoriser la libération du contenu cellulaire au cours de l'extraction. L'étape de traitement par l'acide phytique en milieu basique est essentielle pour enrichir l'extrait en ARN de petits poids moléculaires et également assurer un meilleur rendement d'extraction des autres phytomolécules d'intérêt ; à savoir les sucres, les acides aminés, les composés phénoliques, les acides organiques ainsi que la théine et la théanine.

L'acide phytique utilisé dans le procédé est sous forme de sels de sodium, calcium ou parfois de magnésium.

De manière préférée, l'acide phytique utilisé est une poudre d'acide phytique sous forme de sel de sodium. Préférentiellement on l'utilise à une concentration comprise entre 1 et 10 mM, préférentiellement entre 1 et 5 mM et plus préférentiellement encore à une concentration de 3 mM.

De manière particulièrement avantageuse, on utilise une concentration d'acide phytique sous forme de sel de sodium de 3 mM.

A l'étape c) on ajuste le pH à une valeur basique comprise entre 10 et 11 par l'ajout de soude (NaOH). Lors de l'étape c) il est essentiel que le pH soit basique, compris entre 10 et 11. De préférence, le pH est ajusté à une valeur comprise entre 10,3 et 10,8. En effet, ce niveau de pH, associé à l'action de l'acide phytique, provoque la déstructuration de la membrane cellulaire, y compris la membrane nucléaire, la lyse des cellules végétales et provoque la dénaturation de l'ADN (les 2 brins de la double hélice sont séparés).

L'étape d) d'extraction par lyse alcaline en présence d'acide phytique dure préférentiellement au moins 1h, à une température comprise entre 40 et 80°C. De préférence l'étape dure 1 heure. Avantageusement l'étape est réalisée à une température comprise entre 60°C et 80°C. Encore plus préférentiellement, l'étape est réalisée à 80 °C. Pendant cette étape, le mélange est avantageusement mis sous agitation modérée.

Le contrôle du pH montre que celui-ci reste basique et se stabilise entre 9 et 11 à la fin de l'étape d).

A l'étape e), on purifie le mélange obtenu en d) de manière à éliminer les matières solides résiduelles et récupérer la partie soluble qui constitue l'extrait brut aqueux selon l'invention.

Toute méthode connue par l'homme du métier pourra être utilisée pour réaliser cette étape de purification. De préférence, le mélange est filtré directement sur des filtres de porosité supérieure ou égale à 30 µm de façon à récolter le filtrat. Le mélange obtenu en d) peut également être centrifugé à faible vitesse, par exemple pendant au moins 10 min à 4000 g, de manière à sédimenter la matière végétale résiduelle dans le culot et récupérer l'extrait brut aqueux dans le surnageant.

A l'étape f), on ajuste le pH à une valeur comprise entre 6 et 8. Cette étape d'acidification provoque la renaturation brutale de l'ADN (réappariement des brins de la double hélice). Néanmoins l'ADN chromosomique, très long, ne parvient pas à se réapparier complètement et forme des enchevêtrements insolubles qui seront éliminés ultérieurement. Au contraire, les petits ARN, beaucoup plus courts, restent en solution.

A l'étape g), on ajoute au mélange du charbon actif en poudre. Préférentiellement on utilise un mélange de deux types de charbons différents, dans une proportion de 0,1 à 1 % (poids/poids), et préférentiellement une proportion de 0,25 % (poids/poids) de chacun des deux types de charbons actifs pour accroître la décoloration. Les charbons actifs sont sélectionnés pour leurs capacités à appauvrir l'extrait en polyphénols, qui sont principalement responsables de la couleur de l'extrait. Cette étape dure de 15 min à 1 heure, préférentiellement trente minutes, à une température comprise entre 40 et 50°C sous agitation pour un résultat optimal.

A l'étape h), le mélange obtenu à l'étape g) est filtré pour éliminer les particules de charbon au moyen de filtres de porosité supérieure ou égale à 30 µm. On obtient alors un filtrat clarifié.

A l'étape i), le filtrat obtenu à l'étape h) est mis en contact avec des PVPP en poudre afin de poursuivre l'élimination des composés phénoliques tels que des polyphénols de haut poids moléculaire, types tanins, qui n'auraient pas été retenus par les charbons actifs. De préférence, on utilise les PVPP dans une proportion comprise entre 0,5 %et 3 % (poids/poids), et préférentiellement dans une proportion comprise entre 1 % et 10 %.

Cette étape peut durer entre 10 et 30 min et préférentiellement dix minutes, à une température comprise entre la température ambiante et 50°C, sous agitation.

A l'étape j) le mélange obtenu à l'étape i) est clarifié par filtration sur des filtres de porosité supérieure à 25 µm, suivie d'une filtration utilisant un filtre de 0,8 µm de porosité. Cette étape permet l'élimination des particules de PVPP. On récolte le filtrat.

Le procédé décrit ici et en particulier les étapes g) à j) permet d'obtenir un extrait qui contient des traces, c'est-à-dire au maximum 0,001% de catéchines par rapport au poids sec de l'extrait, voire pas de catéchine, épicatéchine ou épigallocatéchine. Ceci a été démontré par des analyses de l'extrait par chromatographie sur couche mince.

A l'étape k), on ajuste ensuite le pH du mélange obtenu lors de l'étape j) à une valeur comprise entre 6 et 6,5. On obtient alors un extrait brut de thé noir.

Le pH est ajusté par ajout d'une solution d'acide chlorhydrique (HCl) ou tout autre acide équivalent compatible avec une utilisation cosmétique, tel l'acide citrique. L'ajustement du pH entre 6 et 6,5 est une étape indispensable du procédé décrit dans cette demande pour maintenir en suspension les ARN de petit poids moléculaire et prévenir la précipitation des composés phytochimiques d'intérêt tels que les sucres, les composés phénoliques, les acides organiques, les acides aminés, la théine et la théanine.

Le procédé d'extraction décrit ci-dessus permet ainsi d'obtenir un extrait brut aqueux de feuilles de thé noir de l'espèce *Camellia sinensis* ayant un poids sec de 5 à 30 g/kg et comprenant, en poids du poids total de l'extrait, de 0,5 à 10 g/kg de sucres, de 0,050 à 2 g/kg d'acides organiques, de 0,030 à 3 g/kg d'acides aminés, de 0,050 à 5 g/kg de composés phénoliques dont au maximum 150 mg/kg de catéchines, de 0,020 à 2 g/kg de théine, de 0,020 à 2 g/kg de théanine et de 50 à 350 mg/kg d'ARN de petit poids moléculaire d'une longueur d'au maximum 150 nucléotides et est dépourvu d'ADN.

La présente invention a donc également pour objet un extrait brut aqueux de feuilles de thé noir de l'espèce *Camellia sinensis,* susceptible d'être obtenu par le procédé décrit ci-dessus.

L'extrait obtenu à l'étape k) peut optionnellement être dilué avec un solvant physiologiquement acceptable afin d'augmenter sa stabilité et d'augmenter sa préservation dans le temps. On obtient alors un extrait dilué.

L'extrait est dilué de sorte à obtenir un poids sec compris entre 4 et 20 g/kg, son pH est ajusté entre 5,8 et 6,5 préférentiellement entre 6,0 et 6,5. Cette étape permet d'améliorer la stabilité du produit dans le temps.

Le solvant physiologiquement acceptable, peut être choisi parmi les solvants suivants : l'eau, le glycérol, l'éthanol, le propanediol, le butylène glycol, ainsi que leur version naturelle, le dipropylène glycol, les diglycols éthoxylés ou propoxylés, les polyols cycliques ou tout mélange de ces solvants.

De préférence, l'éthanol, le butylène glycol, le propanediol et la glycérine sont des solvants dérivés de plantes (version naturelle).

De manière avantageuse, le solvant physiologiquement acceptable est choisi parmi le butylène glycol, le propanediol ou la glycérine obtenus à partir de plantes.

Le butylène glycol ou le propanediol permet de stabiliser l'extrait et éviter des phénomènes de précipitation des phytocomposés à long terme. La glycérine, lorsqu'elle est utilisée à au moins 70 %, présente les mêmes propriétés. Ces solvants sont aussi reconnus pour leur pouvoir bactériostatique voire bactéricide ou fongicide, qui contribue également à la conservation et la stabilité dans le temps de l'extrait. L'extrait ainsi dilué se conserve pendant au minimum douze mois. Cet aspect a été validé par une étude qui consiste, après inoculation de l'extrait par différents types de microorganismes, à mesurer sa capacité à bloquer la croissance des microorganismes. L'extrait ainsi dilué se conserve pendant au minimum douze mois.

Dans un mode de réalisation particulièrement préféré, l'extrait brut de thé noir peut être mélangé avec 30 à 50 % de butylène glycol, ou 30 à 50 % de propanediol ou encore 30 à 70 % de glycérine.

Lorsque la glycérine est utilisée, elle est de préférence utilisée à 70 %.

Préférentiellement, l'extrait de thé noir est dilué dans du propanediol obtenu à partir de plante de telle sorte que l'extrait dilué comprenne une concentration finale en propanediol de 30 % ou de 50%.

L'extrait aqueux de feuilles de thé noir ainsi dilué a un poids sec compris entre 4 et 20 g/kg et comprend en poids du poids total de l'extrait dilué, de 10 à 250 mg/kg d'ARN de petit poids moléculaire d'une longueur d'au maximum 150 nucléotides, de 0,2 à 5 g/kg de sucres, de 0,030 à 1 g/kg d'acides organiques, de 0,010 à 2 g/kg d'acides aminés, de 0,030 à 3 g/kg de composés phénoliques dont au maximum 120 mg/kg de catéchines, de 0,010 à 1 g/kg de théine, de 0,010 à 1 g/kg de théanine et est dépourvu d'ADN.

Dans un mode de réalisation préféré, le thé noir est d'origine Ile Maurice ou Inde.

L'extrait aqueux de feuilles de thé noir décrit au paragraphe ci-dessus pourra être utilisé directement pour préparer la composition cosmétique de l'invention.

Alternativement, l'extrait brut de thé noir obtenu à l'étape k) peut aussi être déshydraté pour obtenir un extrait sec, sous forme de poudre. Toute méthode connue par l'homme de métier pour déshydrater l'extrait pourra être utilisée, par exemple la lyophilisation.

Un troisième objet de l'invention est une composition cosmétique comprenant en tant qu'agent actif, une quantité efficace d'un extrait dilué de thé noir, obtenu selon le procédé décrit ci-dessus, et un milieu physiologiquement acceptable.

Avantageusement, l'extrait de thé noir est ajouté dans un milieu physiologiquement acceptable à une concentration de 0,05 à 5 % en poids par rapport au poids total de la composition, préférentiellement à une concentration de 0,1 à 2,5 % en poids par rapport au poids total de la composition.

La composition de la présente demande est formulée pour être appliquée par toute voie appropriée, notamment orale, ou topique externe, et la formulation des compositions sera adaptée par l'homme du métier.

Préférentiellement, la composition de la présente demande se présente sous une forme adaptée à l'application par voie topique. Cette composition doit donc contenir un milieu physiologiquement acceptable, c'est-à-dire compatible avec la peau et les phanères, sans risque d'inconfort lors de son application.

La composition pourra notamment se présenter sous forme d'une solution ou d'un gel aqueux, hydroalcooliques ou huileux, d'une émulsion huile-dans-eau, eau-dans-huile ou émulsions multiples ; elles peuvent aussi se présenter sous forme de suspensions, ou encore poudres, adaptées à une application sur la peau, les muqueuses, les lèvres et/ou les cheveux.

La composition peut être plus ou moins visqueuse et avoir également l'aspect d'une crème, d'une lotion, d'un fluide, d'un lait, d'un sérum, d'une pommade, d'un gel, d'une pâte, d'un baume ou d'une mousse. Elle peut aussi se présenter sous forme solide, comme un stick ou être appliquée sur la peau sous forme d'aérosol.

A titre de milieu physiologiquement acceptable communément utilisé dans le domaine d'application envisagé, on peut citer par exemple des adjuvants nécessaires à la formulation, tels que des solvants, des épaississants, des gélifiants, des diluants, des émulsionnants, des antioxydants, des colorants, des filtres solaires, des agents auto-bronzants, des pigments, des charges, des conservateurs, des parfums, des absorbeurs d'odeur, des huiles essentielles, des vitamines, des acides gras essentiels, des tensioactifs, des polymères filmogènes, des esters, des huiles ou beurres végétaux, etc.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre chacun à 0,01 à 20 % du poids total de la composition. Lorsque la composition selon l'invention est une émulsion, la phase grasse peut représenter de 2 à 90 % en poids et de préférence de 5 à 30 % en poids par rapport au poids total de la composition. Les émulsionnants et co-émulsionnants utilisés dans la composition sont choisis parmi ceux classiquement utilisés dans le domaine considéré. Par exemple, ils peuvent être utilisés en une proportion allant de 0,3 à 30 % en poids par rapport au poids total de la composition.

Selon un autre mode de réalisation avantageux, l'extrait de thé noir peut être encapsulé ou inclus dans un vecteur cosmétique tels que les liposomes ou toute autre nano capsule ou microcapsule utilisée dans le domaine de la cosmétique ou adsorbé sur des polymères organiques poudreux, des supports minéraux comme les talcs et bentonites.

Avantageusement, la composition peut comprendre, outre l'agent actif, c'est-à-dire un extrait de thé noir, au moins un autre agent actif présentant des effets cosmétiques similaires et/ou complémentaires à ceux de l'invention.

Par exemple, le ou les agents actifs additionnels peuvent être choisis parmi : les agents anti-âge, raffermissants, éclaircissants, hydratants, drainants, favorisant la microcirculation, exfoliants, desquamants, stimulant la matrice extracellulaire, activant le métabolisme énergétique, antibactériens, antifongiques, apaisants, anti-radicalaires, anti-UV, anti-acné, anti-inflammatoires, anesthésiques, procurant une sensation de chaleur, procurant une sensation de fraîcheur, amincissants.

De tels agents actifs additionnels peuvent être choisis dans les groupes comprenant :
- la vitamine A et notamment l'acide rétinoïque, le rétinol, le rétinol propionate, le rétinol palmitate ;
- la vitamine B3 et plus particulièrement le niacinamide, le nicotinate de tocophérol ;
- la vitamine B5, la vitamine B6, la vitamine B12, le panthénol ;
- la vitamine C, notamment l'acide ascorbique, l'ascorbyl glucoside, l'ascorbyl tétrapalmitate, magnésium et sodium ascorbyl phosphate ;
- les vitamines E, F, H, K, PP, le coenzyme Q10 ;
- les inhibiteurs de métalloprotéinase, ou un activateur des TIMP ;
- la DHEA, ses précurseurs et dérivés ;
- les acides aminés tels que l'arginine, ornithine, hydroxyproline, hydroxyproline dipalmitate, palmitoylglycine, hydroxylysine, méthionine et ses dérivés, composés acides aminés N-acylés ;
- les peptides naturels ou de synthèse, incluant les di-, tri-, tetra-, penta- et hexapeptides et leurs dérivés lipophiles, isomères et complexés avec d'autres espèces tel qu'un ion métal (par exemple cuivre, zinc, manganèse, magnésium, et autres). A titre d'exemples, on peut citer les peptides commercialement connus sous les noms de MATRIXYL^{®}, ARGIRELINE^{®}, CHRONOGEN^{™}, LAMINIXYL IS^{™}, PEPTIDE Q10^{™}, COLLAXYL ^{™} (brevet FR2827170, ASHLAND^{®}), PEPTIDE VINCI 01^{™} (brevet FR2837098, ASHLAND^{®}), PEPTIDE VINCI 02^{™} (brevet FR2841781, ASHLAND^{®}), ATPeptide^{™} (brevet FR2846883, ASHLAND^{®}) ou encore le peptide de synthèse de séquence Arg-Gly-Ser-NH2, commercialisé sous le nom ATPeptide^{™} par ASHLAND^{®} ;
- l'extrait d'Artémia salina, commercialisé sous le nom GP4G^{™} (FR2817748, ASHLAND^{®}) ;
- les extraits peptidiques végétaux tels que les extraits de lin (Lipigénine^{™}, brevet FR2956818, ASHLAND^{®}), les extraits de soja, d'épeautre, de vigne, de colza, de lin, de riz, de maïs, de pois ;
- les extraits de levures, par exemple le Dynagen^{™}, (brevet FR2951946, ASHLAND^{®}) ou l'Actopontine^{™} (brevet FR2944526, ASHLAND^{®}) ;
- l'acide déhydroacétique (DHA) ;
- les phytostérols d'origine synthétique ou naturelle ;
- l'acide salicylique et ses dérivés, les alpha- et bêta-hydroxyacides, les silanols ;
- les sucres aminés, glucosamine, D-glucosamine, N-acetyl glucosamine, N-acetyl-D-glucosamine, mannosamine, N-acetyl mannosamine, galactosamine, N-acetyl galactosamine ;
- les extraits de polyphénols, isoflavones, flavonoïdes, tels que les extraits de raisin, les extraits de pin, les extraits d'olive ;
- les lipides tels que les céramides ou les phospholipides, les huiles d'origine animale, telles que le squalène ou le squalane ; les huiles végétales, telles que l'huile d'amande douce, de coprah, de ricin, de jojoba, d'olive, de colza, d'arachide, de tournesol, de germes de blé, de germes de maïs, de soja, de coton, de luzerne, de pavot, de potiron, d'onagre, de millet, d'orge, de seigle, de carthame, de passiflore, de noisette, de palme, de noyau d'abricot, d'avocat, de calendula ; les huiles végétales éthoxylées, les beurres de karité, de cacao, de babassu ;
- tous écrans UV et filtres solaires ;
- l'AMP cyclique et ses dérivés, les agents activateurs de l'enzyme adénylate cyclase et les agents inhibiteurs de l'enzyme phosphodiestérase, l'extrait de Centella asiatica, l'asiaticoside et l'acide asiatique, les méthyls xanthines, la théophylline, la théobromine, la forskoline, l'esculine et l'esculoside, les inhibiteurs d'ACE, le peptide Val-Trp, l'inhibiteur du neuropeptide Y, l'enkephaline, l'extrait de Ginkgo biloba, l'extrait de dioscorea, la rutine, l'extrait de yerba mate, l'extrait de guarana, les oligosaccharides, les polysaccharides, la carnitine, l'extrait de lierre, l'extrait de fucus, l'extrait hydrolysé de Prunella vulgaris, l'extrait hydrolysé de Celosia cristata, l'extrait d'Anogeissus leiocarpus, l'extrait de feuilles de Manihot utilissima, la palmitoylcarnitine, la carnosine, la taurine, l'extrait de sureau, les extraits d'algues tel que l'extrait de Palmaria Palmata ;
- l'acide hyaluronique ou sodium hyaluronate ainsi que toutes ses fractions et promoteurs ;
- le menthol et menthyl lactate et autres actifs à effet frais ;
- l'aloe vera, le bisabolol, l'allantoin et autres actifs à effet apaisant ;
- le Vanillyl Butyl Ether et autres actifs à effet chauffant ;
- le Benzoyl Peroxide et autres actifs anti-acné.

Dans un mode de réalisation particulier de l'invention, la composition cosmétique comprend, en tant qu'agent actif, une quantité efficace d'un extrait de thé noir de toute provenance, à l'exclusion du thé noir provenant de l'Ile Maurice, obtenu selon le procédé décrit ci-dessus, et un milieu physiologiquement acceptable.

L'invention a pour quatrième objet l'utilisation cosmétique d'une composition décrite précédemment comprenant un extrait de thé noir de toute provenance à l'exclusion du thé noir provenant de l'Ile Maurice, pour le soin de la peau, du cuir chevelu et des phanères, et plus particulièrement pour protéger la peau des agressions externes et de l'oxydation, lutter contre les signes du vieillissement cutané, augmenter la photoprotection, éclaircir la peau, améliorer l'hydratation de la peau.

Le terme « utilisation cosmétique » signifie que l'utilisation est prévue pour des individus ayant une peau, un cuir chevelu ou des phanères sains.

La peau est un organe composé de plusieurs couches (derme, épiderme et stratum corneum), qui recouvre toute la surface du corps et assure des fonctions protectrices vis-à-vis des agressions externes, sensitives, immunitaires, métaboliques, thermorégulatrices ou encore de barrière limitant la déshydratation.

L'aspect de la peau peut être modifié par des altérations internes (vieillissement intrinsèque, maladies et changements hormonaux tels que la grossesse) ou externes (facteurs environnementaux, tels que la pollution, la lumière du soleil, les pathogènes, les variations de température, etc.). Toutes ces altérations affectent non seulement la peau, mais aussi les annexes kératiniques ou phanères telles que les poils, les cils, les sourcils, les ongles et les cheveux.

### Exemples

A titre illustratif, des exemples de mode de réalisation du procédé selon l'invention sont décrits ci-dessous.

### Exemple 1 : Préparation d'extraits de thé noir (Camellia sinensis) provenant d'Inde et d'Ile Maurice enrichis en petits ARN selon le procédé de l'invention :

Afin de tenir compte de la grande capacité d'adaptation de la plante à son environnement, des extraits de thés noirs (tous issus de l'espèce *Camellia sinensis*) de différentes origines géographiques ont été utilisés pour mettre en œuvre le procédé de l'invention tel que décrit dans cet exemple.

Des extraits de thé noir provenant de l'Ile Maurice et de thé noir provenant d'Inde, ont été préparés.

L'île Maurice est surmontée de chaînes de montagnes, dont la hauteur varie de 300 à 800 m au-dessus du niveau de la mer. La terre monte des plaines côtières à un plateau central où elle atteint une hauteur de 670 m. Le climat de l'île en fait un lieu idéal pour la culture du thé. Le thé noir provenant de l'île Maurice désigne un thé cultivé, récolté et transformé dans des plantations situées sur l'Ile Maurice.

Le thé d'Inde désigne un thé cultivé, récolté et transformé dans des plantations de thé situés entre 1200 à 2200 m d'altitude en Inde.

Dans une première étape a) les feuilles de thé noir, préalablement broyées sous forme de poudre grossière, sont pesées pour représenter 3 % de matière première engagée dans le procédé, soit 30 g pour 1 kg. La quantité d'eau distillée ajoutée est de 968 g.

Le mélange feuilles de thé et eau est mis sous agitation pour permettre une meilleure extraction des phytomolécules d'intérêt grâce à un meilleur échange entre la matière solide, le thé, et la solution aqueuse d'extraction.

Dans une étape b), de l'acide phytique à 2 g/l (soit 3 mM final) est ajouté aux mélanges eau et feuilles de thé broyées.

Dans une étape c), le pH est ajusté à 10,5 pour que l'extraction soit optimale et permettre un enrichissement de l'extrait en ARN de petits poids moléculaires ainsi qu'en diverses phytomolécules.

Dans une étape d) le mélange est chauffé 1h à 80°C sous agitation modérée.

Dans une étape e) le mélange est filtré à l'aide de filtres de porosité de 30 µm, pour séparer la matière solide du filtrat. Trois filtrations séquentielles sont ensuite réalisées à l'aide de filtres de porosité décroissante afin de clarifier l'extrait végétal jusqu'à une filtration à 3 µm de porosité.

Etape f) Le pH du filtrat récolté à l'étape e) supérieur à 9 en moyenne est ajusté entre 6 et 8 pour se placer à un pH optimal pour le traitement aux charbons actifs qui suit.

Etape g) Un traitement par un duo de charbons actifs NORIT^{®} SX+ et CA1, est effectué afin de décolorer l'extrait en ôtant notamment des polyphénols. 2,5 g de chaque type de charbon par litre d'extrait sont ajoutés. La durée de traitement est de trente minutes à une température comprise entre 40 et 50°C.

Etape h) : Les charbons sont éliminés en passant l'extrait sur des filtres de porosité de 30 µm.

A l'étape i) un second traitement de décoloration du filtrat obtenu en g) est effectué en ajoutant des (PVPP) en poudre. 10 g de PVPP sont ajoutés pour un litre d'extrait afin de retirer une partie des tanins contenus dans l'extrait. La durée de traitement est de 10 minutes à une température comprise entre 40 et 50°C.

A l'étape j) des filtrations séquentielles sur filtres de porosité décroissante sont alors réalisées afin de retirer le PVPP de l'extrait végétal, une première filtration de 30 µm permet de retenir le PVPP suivi de filtrations successives allant jusqu'à une porosité de 0,8 µm afin de clarifier l'extrait.

A l'étape k) le pH est contrôlé, puis ajusté à une valeur comprise entre 6 et 6,5 avec une solution d'acide citrique ou d'acide chlorhydrique.

L'extrait de thé noir brut aqueux ainsi obtenu à partir de thé noir provenant de l'Ile Maurice a un poids sec de 10,8 g/kg. L'analyse physico-chimique montre que l'extrait obtenu présente une concentration de 3,3 g/kg de sucres totaux, 550 mg/kg d'acides organiques totaux, 340 mg/kg d'acides aminés, 1,2 g/kg de composés phénoliques totaux et 260 mg/kg d'ARN de petits poids moléculaires d'une longueur d'au maximum 150 nucléotides.

L'extrait de thé noir brut aqueux obtenu à partir de thé noir provenant d'Inde à un poids sec de 7,8 g/kg. L'analyse physico-chimique montre que l'extrait obtenu présente une concentration de 1,2 g/kg de sucres totaux, 280 g/kg d'acides organiques totaux, 230 mg/kg d'acides aminés, 0,51 g/kg de composés phénoliques totaux et 160 mg/kg d'ARN de petits poids moléculaires d'une longueur d'au maximum 150 nucléotides.

Les extraits obtenus à l'étape k) sont dilués avec du propanediol dérivé de plante, de manière à obtenir une concentration finale de 30 % de propanediol et 70 % d'extrait de thé. Ce solvant physiologiquement acceptable pour un usage cosmétique permet de stabiliser le produit et d'augmenter sa préservation dans le temps. On obtient alors un extrait dilué dont on ajuste le pH entre 6,0 et 6,5.

La composition des extraits aqueux bruts après ajout de 30 % de propanediol est donnée dans le tableau 1 ci-dessous.

**[Tableau 1]**

| | **Extrait PSR thé noir Ile Maurice** | **Extrait PSR thé noir Inde** |
|---|---|---|
| Poids sec (g/Kg) | 7,4 | 5,6 |
| Sucres totaux (g/kg) | 1,2 | 0,5 |
| Acides organiques totaux (mg/kg) | 310 | 155 |
| Acides aminés (mg/kg) | 262 | 120 |
| Composés phénoliques totaux (mg/kg) | 770 | 310 |
| ARN d'une longueur d'au maximum 150 nucléotides (mg/kg) | 154 | 85 |

Les extraits de thé dilués ainsi obtenus selon le procédé décrit dans cet exemple sont appelés PSR (Plant Small RNA) thé dans les tableaux ou figure présentée dans la présente demande.

### Exemple 2 : Préparation d'un extrait comparatif de thé noir (Camellia sinensis) par une méthode d'extraction dite conventionnelle :

Le procédé mis en œuvre dans cet exemple 2 a pour but de préparer un extrait contrôle pour obtenir des données analytiques comparatives par rapport aux extraits de thé obtenus par le procédé de l'invention (exemple 1).

La méthode d'extraction dite conventionnelle choisie est optimale pour extraire les différents types de composés phénoliques et autres molécules polaires comme les sucres, les acides aminés ce qui en fait un bon procédé de référence pour réaliser des comparaisons avec le procédé de l'invention appliqué dans l'exemple 1.

Dans une première étape pour fabriquer 1 kg d'extrait, les mêmes thés que dans l'exemple 1, provenant de l'Ile Maurice et d'Inde ont été utilisés (issus de l'espèce *Camellia sinensis).*

Les feuilles de thés noir séchées, préalablement broyées sous la forme de poudre grossière, sont pesées pour représenter 3 % de matière première engagée dans le procédé soit 30 g pour 1 kg auquel on mélange à 970 g d'eau distillée.

Le pH d'extraction n'est pas ajusté et se situe entre 5 et 7.

Le mélange est ensuite chauffé 1h à 45°C sous agitation.

Le mélange est ensuite filtré à l'aide de filtres de porosité de 30 µm, pour séparer la matière solide du filtrat. Des filtrations séquentielles sur filtres de porosité décroissante sont alors réalisées afin de clarifier l'extrait végétal jusqu'à une filtration à 1 µm de porosité.

A l'issue de cette étape, un traitement au charbon est effectué afin de décolorer l'extrait en ôtant notamment des polyphénols. 2,5 g de chaque charbon par litre d'extrait est ajouté. La durée de traitement est de trente minutes à une température comprise entre 40 et 50°C et à un pH compris entre 6 et 8. Une filtration de 30 µm est réalisée pour ôter les charbons de l'extrait.

Puis un second traitement de décoloration est effectué en ajoutant des PVPP en poudre. 10 g de PVPP sont ajoutés à l'extrait afin de retirer une partie des tanins contenus dans l'extrait. La durée de traitement est de 10 minutes à une température comprise entre 40 et 50°C. Des filtrations séquentielles sur filtres de porosité décroissante sont alors réalisées afin de clarifier l'extrait végétal jusqu'à une filtration à 0,2 µm.

Le pH est contrôlé, puis il est ajusté entre 6 et 6,5 avec une solution d'acide citrique ou d'acide chlorhydrique ou de soude.

L'extrait de thé noir brut provenant de l'Ile Maurice obtenu a un poids sec de 7 g/kg. L'analyse physico-chimique montre que l'extrait obtenu présente une concentration de 0,9 g/kg de sucres totaux, 310 mg/kg d'acides organiques totaux, 190 mg/kg d'acides aminés, 270 mg/kg de composés phénoliques totaux.

L'extrait de thé noir brut aqueux provenant d'Inde obtenu a un poids sec de 4 g/kg. L'analyse physico-chimique montre que l'extrait obtenu présente une concentration de 0,8 g/kg de sucres totaux, 190 mg/kg d'acides organiques totaux, 140 mg/kg d'acides aminés, 170 mg/kg de composés phénoliques totaux.

L'extrait est ensuite dilué avec du propanediol dérivé de plante, un solvant cosmétique physiologiquement acceptable, de manière à obtenir une concentration finale de 30 % de propanediol et 70 % d'extrait de thé. La composition des extraits obtenus par la méthode conventionnelle est donnée dans le tableau 2 ci-dessous.

**[Tableau 2]**

| | **Extrait conventionnel thé noir Ile Maurice** | **Extrait conventionnel thé noir Inde** |
|---|---|---|
| Poids sec (g/Kg) | 5 | 2,8 |
| Sucres totaux (g/kg) | 0,5 | 0,4 |
| Acides organiques totaux (mg/kg) | 210 | 132 |
| Acides aminés (mg/kg) | 112 | 80 |
| Composés phénoliques totaux (mg/kg) | 180 | 120 |
| ARN d'une longueur d'au maximum 150 nucléotides (mg/kg) | nd | nd |

### Exemple 3 : Quantification des différents composants des extraits dilués de thé noir obtenus dans les exemples 1 et 2 et évaluation de la stabilité microbiologique de l'extrait de l'exemple 1

Le poids sec de chaque extrait, obtenu selon l'exemple 1 (procédé de l'invention) ou selon l'exemple 2 (procédé conventionnel) a été mesuré après 12h d'étuvage à 105°C.

Les extraits PSR obtenus par le procédé de l'exemple 1 (procédé de l'invention) ont un poids sec supérieur à celui des extraits de thé obtenus par le procédé conventionnel, ce qui signifie que le rendement d'extraction est supérieur dans le procédé de l'exemple 1 (voir tableau 3).

**[Tableau 3]**

| | **Extrait PSR thé noir Ile Maurice** | **Extrait PSR thé noir Inde** | **Extrait conventionne** I **thé noir Ile Maurice** | **Extrait conventionne** I **thé noir Inde** |
|---|---|---|---|---|
| Poids sec (g/Kg) | 7,4 | 5,6 | 5 | 2,8 |
| Rendement d'extraction (%) | 26 | 19 | 16 | 9,3 |

La teneur totale en sucres des extraits de thés des exemples 1 et 2 a été déterminée par dosage spectrophotométrique issu d'une adaptation du dosage décrit par Dubois et al. (1956) (Dubois et al., "Méthode colorimétrique pour la détermination des sucres et des substances apparentées", Anal. Chem., 1956, 28 (3), 350-356). Les extraits de thé noir des exemples 1 et 2 sont dissous dans l'acide sulfurique concentré puis réagissent avec du phénol pour former un complexe coloré. L'absorbance du complexe est lue sur le spectrophotomètre à 490 nm. La teneur en sucre est déterminée à l'aide d'une courbe standard de glucose.

L'analyse met en évidence une plus forte concentration en sucres dans les extraits de thé noir de toutes provenances, obtenus par le procédé de l'exemple 1 comparativement à la concentration en sucre de l'extrait obtenu par la méthode conventionnelle de l'exemple 2. Les résultats sont illustrés dans le tableau 4.

**[Tableau 4]**

| | **Extrait PSR thé noir Ile Maurice** | **Extrait PSR thé noir Inde** | **Extrait conventionn el thé noir Ile Maurice** | **Extrait conventionn el thé noir Inde** |
|---|---|---|---|---|
| Sucres totaux (g/kg) | 1,2 | 0,5 | 0,5 | 0,4 |

La teneur totale en acides aminés des extraits de thé noir des exemples 1 et 2 a été déterminée par dosage spectrophotométrique à partir d'un protocole publié par Moore S. et Stein WH. (1948) (Moore, S. and Stein, W.H. (1948) Photometris Ninhydrin Method for Use in the Chromatography of Amino Acids. The Journal of Biological Chemistry, 176, 367-388).

La teneur en acides aminés libres de l'extrait a été évaluée par la formation d'un complexe coloré, à la suite de la rupture des fonctions amine et carboxylique par le réactif ninhydrine. L'absorbance du complexe a été lue à 570 nm. La teneur totale en acides aminés a été déterminée en utilisant une courbe standard réalisée à partir d'un pool d'acides aminés.

L'analyse met en évidence une plus forte concentration en acides aminés dans les extraits PSR de thé noir obtenus par le procédé de l'invention de l'exemple 1 comparativement à la concentration en acides aminés des extraits obtenus par la méthode conventionnelle de l'exemple 2.

**[Tableau 5]**

| | **Extrait PSR thé noir Ile Maurice** | **Extrait PSR thé noir Inde** | **Extrait conventionnel thé noir Ile Maurice** | **Extrait conventionnel thé noir Inde** |
|---|---|---|---|---|
| Acides aminés totaux (mg/kg) | 262 | 120 | 112 | 80 |

La teneur totale en composés phénoliques contenue dans les extraits de thé noir des exemples 1 et 2 a été déterminée par la méthode spectrophotométrique de Folin-Ciocalteu (Singleton et al., Analyse des phénols totaux et d'autres substrats d'oxydation et antioxydants au moyen du réactif Folin-Ciocalteu, 1999, 299: 152). Les composés de type polyphénols présents dans l'échantillon réagissent avec le réactif de Folin-Ciocalteu pour donne une couleur bleue. L'absorbance de l'échantillon est lue sur le spectrophotomètre à 760 nm. La concentration totale en polyphénol est exprimée en équivalents d'acide gallique à l'aide d'une courbe standard d'acide gallique.

L'analyse met en évidence une plus forte concentration de composés phénoliques dans les extraits obtenus grâce au procédé décrit dans l'invention pour toutes les origines de thés noirs comparativement avec le procédé d'extraction conventionnel, comme décrit dans le tableau 6.

**[Tableau 6]**

| | **Extrait PSR thé noir Ile Maurice** | **Extrait PSR thé noir Inde** | **Extrait conventionnel thé noir Ile Maurice** | **Extrait conventionnel thé noir Inde** |
|---|---|---|---|---|
| Composés phénoliques totaux (mg/kg) | 770 | 310 | 180 | 120 |
| % (poids/poids) Composés phénoliques totaux / poids sec extrait | 13 | 5,5 | 5 | 4,2 |

La caractérisation et la quantification des acides organiques contenus dans les extraits de thé noir des exemples 1 et 2 a été réalisée par chromatographie liquide haute performance, couplée à un détecteur de masse ACQUITY Qda (WATERS). Les échantillons ont été séparés sur une colonne EC 150 / 4,6 Nucleoshell RP 18plus-5µm (150x4,6 mm) (Macherey Nagel: 763236.46) par un système HPLC Agilent 1260 (Agilent Technologies). Le débit était de 0,3 ml / min. La phase mobile consistait en une solution d'acide formique (HCOOH) à 0,01 % (A) et d'acétonitrile.

L'identification des acides organiques a été réalisée en comparant les temps de rétention et les pics spectraux de masse de l'échantillon avec un étalon. L'estimation quantitative des acides organiques a été réalisée sur la base de l'aire des pics chromatographiques comparée aux aires des étalons.

L'analyse HPLC-MS montre ainsi que les extraits de thé noir obtenus selon le procédé de l'invention provenant d'Ile Maurice ou d'Inde ont une concentration totale en acides organiques plus forte que les extraits conventionnels décrits dans l'exemple 2, tels que décrit dans le tableau 7.

L'analyse montre également que les extraits de thé noir obtenus selon les exemples 1 et 2 contiennent différents types d'acides organiques, tel qu'illustré dans le tableau 7. L'acide tartrique et les acides uroniques n'ont pas été détectés ou en quantité très faible que ce soit dans les extraits PSR de thé noir obtenus selon l'exemple 1 ou dans les extraits conventionnels de l'exemple 2.

**[Tableau 7]**

| Type d'extraits | **Acide lactique (mg/kg)** | **Acide succiniqu e (mg/kg)** | **Acide malique (mg/kg)** | **Acide Tartrique (mg/kg)** | **Acide uronique (mg/kg)** |
|---|---|---|---|---|---|
| PSR Thé noir Ile Maurice | 143,3 | 6,9 | 57,9 | nd | 14 |
| PSR Thé noir Inde | 4,6 | 9,9 | 47,2 | 4,2 | nd |
| Extrait conventionnel thé noir Ile Maurice | 3,3 | 8,4 | 54 | nd | nd |
| Extrait conventionnel thé noir Inde | 17,1 | 3,5 | 34,6 | nd | 13,8 |

L'évaluation de la teneur en théanine des extraits de thé noir des exemples 1 et 2 a été réalisée par chromatographie liquide haute performance, couplée à un détecteur UV (lumière ultraviolette). Les échantillons ont été séparés sur une colonne Uptisphère C18-2 250 mm x 4,6 mm x 5 um (Interchim) par un système HPLC Agilent 1200 (Agilent Technologies). Le débit était de 0,4 ml / min. La phase mobile était constituée de 0,1 % d'acide phosphorique (H3PO4) en solution aqueuse (A) et d'acétonitrile (ACN) (B)

L'identification de la théanine a été réalisée par comparaison du temps de rétention et du pic spectral UV de l'échantillon avec un standard. Une estimation quantitative de la théanine a été effectuée sur la base de l'aire des pics chromatographiques comparée à l'aire de l'étalon.

L'analyse HPLC-UV montre que les extraits de thé noir obtenus selon l'exemple 1 contiennent de la théanine en quantité équivalente, et contiennent d'avantage que les extraits conventionnels. Les résultats sont représentés sur le tableau 8.

**[Tableau 8]**

| | **Extrait PSR thé noir Ile Maurice** | **Extrait PSR thé noir Inde** | **Extrait conventionnel de thé noir ile Maurice** | **Extrait conventionnel thé noir Inde** |
|---|---|---|---|---|
| L-théanine (mg/kg) | 54 | 51 | 44 | 41 |

L'évaluation de la teneur en théine (ou caféine) des extraits de thé noir des exemples 1 et 2 a été réalisée par chromatographie liquide haute performance, couplée à un détecteur UV. Les échantillons ont été séparés sur une colonne Uptisphere CS evolution C18-AQ de 100 mm x 4,6 mm x 2,6 pm (Interchim) par un système HPLC Agilent 1100 (Agilent Technologies). Le débit était de 0,8 ml / min. La phase mobile était constituée de 0,1 % de TFA (acides trifluoroacétiques) en solution aqueuse (A) et de méthanol (B).

La température était de 25° C. Le volume d'injection était de 5 µL et la longueur d'onde de détection a été réglée à 272 nm en utilisant un détecteur UV. L'étalon de théine a été acheté chez Sigma-Aldrich. L'identification de la théine a été réalisée par comparaison des temps de rétention et des pics spectraux UV de l'échantillon avec un standard authentique. Une estimation quantitative de la théine a été effectuée sur la base de l'aire des pics chromatographiques comparée à l'aire de l'étalon.

L'analyse HPLC-UV montre que tous les types d'extraits de thé contiennent de la théine. Les extraits de thé noir obtenus par l'extraction conventionnelle mentionnée dans l'exemple 2 ont une concentration en théine supérieure par rapport aux extraits obtenus selon le procédé objet de l'invention. Les résultats sont présentés dans le tableau 9.

**[Tableau 9]**

| | **Extrait PSR thé noir Ile Maurice** | **Extrait PSR thé noir Inde** | **Extrait conventionnel de thé noir Ile Maurice** | **Extrait conventionnel thé noir Inde** |
|---|---|---|---|---|
| théine (mg/kg) | 68 | 122 | 80 | 164 |
| % théine/ poids sec extrait | 0,9 | 2,2 | 5,3 | 2,1 |

La quantification des catéchines totales a également été réalisée. Les catéchines existent sous forme de plusieurs stéréo-isomères due à la présence de deux carbones asymétriques. Dans la nature, les isomères les plus fréquents sont la (+)-catéchine et la (-)-épicatéchine. Les deux autres énantiomères sont beaucoup plus rares et leur présence semble liée à des réactions enzymatiques ou à des traitements thermiques. Tous les échantillons ont été séparés sur une colonne Uptisphere CS evolution C18-AQ de 100 mm x 4,6 mm x 2,6 pm (Interchim) par un système HPLC Agilent 1100 (Agilent Technologies). Le débit était de 0,8 ml / min. La phase mobile était constituée de 0,1 % de TFA (acides trifluoroacétiques) en solution aqueuse (A) et de méthanol (B).

L'analyse HPLC-UV qui permet de quantifier et d'identifier les catéchines contenues dans les différents extraits de thé noir obtenus selon les exemples 1 et 2 (procédé de l'invention et procédé dit conventionnel), montre que les extraits de thé noir obtenus soit par le procédé de l'invention soit par l'extraction conventionnelle contiennent des traces c'est-à-dire au maximum 0,001% de polyphénols de type catéchines par rapport au poids sec de l'extrait voire pas de polyphénols de type catéchines tel qu'illustré dans le tableau 10 (nd signifie non déterminable). Cette absence ou la faible quantité de ces molécules dans les extraits s'explique notamment par la réalisation des étapes de traitement par les charbons et par le PVPP qui ont ôté ce type de composé. Ceci confirmant l'efficacité du traitement charbon associé au PVPP pour ôter ce type de molécule.

**[Tableau 10]**

| | **Extrait PSR thé noir Ile Maurice** | **Extrait PSR thé noir Inde** | **Extrait conventionnel de thé noir Ile Maurice** | **Extrait conventionnel thé noir Inde** |
|---|---|---|---|---|
| catéchines (mg/kg) | nd | 28 | nd | nd |
| % catéchines / poids sec l'extrait | 0 | 0,001 | 0 | 0 |

De plus, des analyses par chromatographie sur couche mince ont montré qu'aucun des extraits de thé de l'invention ne contenaient d'épicatéchine ou d'épigallocatéchine.

La quantification et la mesure de la taille des ARN de bas poids moléculaires ont été réalisées par une technique d'électrophorèse miniaturisée sur des puces microfluidiques spécifiques de l'analyse des acides nucléiques telle que celle des ARN de petits poids moléculaires (Bioanalyseur 2100^{®}, Agilent). Cette méthode permet de déterminer la taille et la concentration en acides nucléiques contenues dans un extrait.

Les petits ARN présents dans les extraits de thé de l'invention sont sous la forme d'un mélange complexe d'ARN ayant une taille majoritairement comprise entre 25 et 150 nucléotides.

Les résultats de la quantification par le Bioanalyseur montrent que les extraits de thé noir obtenus par le procédé de l'exemple 1, permettent d'extraire les ARN de petits poids moléculaires à partir des différents types de thé tel qu'illustré dans le tableau 11. A l'inverse, aucun ARN de petits poids moléculaires n'est détectable dans les extraits de thé noir conventionnels (nd signifie non déterminable).

**[Tableau 11]**

| | **Extrait PSR thé noir Ile Maurice** | **Extrait PSR thé noir Inde** | **Extrait conventionnel thé noir ile Maurice** | **Extrait conventionnel thé noir Inde** |
|---|---|---|---|---|
| Petits ARN (mg/Kg) | 154 | 85 | nd | nd |
| Petits ARN (% total extrait sec) | 2,1 | 1,5 | 0 | 0 |

Evaluation de la stabilité microbiologique de l'extrait de thé noir selon l'exemple 1

La stabilité d'un extrait de thé noir d'origine Inde obtenu selon l'exemple 1 a été réalisé selon le protocole suivant :

L'inoculation a été réalisée au jour 0 et au jour 21. Les tests ont été réalisés 48 h, 7 jours, 14 jours, 21 jours et 28 jours après l'inoculation. Le milieu de culture pour les bactéries était de l'agar tryptone-soja et pour les champignons de l'agar dextrose-pomme de terre.

Des conditions de températures et de temps stantards, adaptés aux types de microorganismes testés, ont été utilisés.

### Résultats

L'extrait de thé noir origine Inde brut (non dilué) présente un taux de contamination supérieur à 100 CFU après quelques jours.

L'extrait de thé noir origine Inde dilué avec du propanediol, de manière à obtenir une concentration finale de 30 % de propanediol et 70 % d'extrait de thé donne les résultats présentés dans le tableau 12.

**[Tableau 12]**

| Microorganisme | Inoculum (cfu/g) | 48h | 7 jours | 14 jours | 21 jours | 28 jours |
|---|---|---|---|---|---|---|
| *Staphylococcus aureus* | 4,5 X 10⁶ | < 10 | < 10 | < 10 | < 10 | < 10 |
| *Escherichia col,* | 2,0 X 10⁶ | < 10 | < 10 | < 10 | < 10 | < 10 |
| *Pseudomonas aeruginosa* | | | | | | |
| *Burkholderia cepacia* | | | | | | |
| *Candida albicans* | 2,5 X 10⁵ | < 10 | < 10 | < 10 | < 10 | < 10 |
| *Aspergillus brasiliensis* | | | | | | |

### Exemple 4 : Evaluation des extraits de thé noir des exemples 1 et 2 sur leur activité vis-à-vis de l'inhibition d'une enzyme, la hyaluronidase en test in vitro:

### Principe :

L'objectif de cet essai est de démontrer le pouvoir inhibiteur des extraits issus des exemples 1 et 2 de provenance Inde sur l'activité enzymatique de la hyaluronidase en utilisant un test de turbidité. L'acide hyaluronique est un glycosaminoglycane présent dans le derme, capable de retenir une grande quantité d'eau dans la matrice extracellulaire dermique et ce faisant est un acteur majeur de l'hydratation cutanée. La hyaluronidase est une enzyme présente dans la peau qui catalyse la dégradation de l'acide hyaluronique, en mono ou disaccharides ainsi qu'en fragments plus petits d'acide hyaluronique. En présence d'albumine acide, l'acide hyaluronique a la capacité de réagir pour former un trouble qui peut être mesuré en spectrophotomètrie à 600 nm. La mesure de l'inhibition de l'activité enzymatique se fait donc en analysant les niveaux de turbidité des échantillons après les avoir mis en contact avec l'enzyme et son substrat.

### Protocole :

L'enzyme est incubée avec les extraits obtenus selon les exemples 1 et 2 origines Inde, de manière à ce que l'extrait soit à une concentration finale de 1 % dans le mélange réactionnel, pendant 20 minutes à une température de 37°C, dans un tampon à pH7, conditions idéales pour l'équilibre enzymatique. Le substrat de l'enzyme, l'acide hyaluronique est ajouté au mélange à une concentration de 0,3 mg/ml. L'ensemble est homogénéisé lentement puis, incubé pendant 45 minutes à 37°C. L'acide hyaluronique restant dans le mélange est ensuite mis en contact avec une solution d'albumine acide et homogénéisé. Le temps réactionnel est de 10 minutes, par la suite la transmittance est lue à 600 nm au spectrophotomètre. Les données sont comparées à celles obtenues avec un contrôle négatif d'inhibition correspondant à la condition où l'enzyme a dégradé à 100 % le substrat, correspondant ainsi à une activité enzymatique de 100 %.

### Résultats :

L'extrait de thé noir provenance Inde préparé selon l'exemple 1 et testé à 1 % montre un pourcentage d'inhibition de la hyaluronidase de 45,1 %. L'extrait de thé origine Inde préparé de manière conventionnelle selon l'exemple 2 montre des pourcentages d'inhibition de 22,1 %. Les résultats sont illustrés par la figure 1.

### Conclusion :

Les essais d'évaluation de l'effet inhibiteur d'extraits testés à 1 % sur l'activité de la hyaluronidase *in vitro* démontrent que l'extrait de thé noir préparé selon l'exemple 1 (procédé de l'invention) provenance Inde présente un pouvoir inhibiteur de l'enzyme significativement plus fort que l'extrait préparé selon un procédé conventionnel (exemple 2)

### Exemple 5 : Formule d'un masque anti-âge

**[Tableau 13]**

| Ingrédients (Nom de marque) | INCI | % w/w |
|---|---|---|
| Phase A | | |
| Eau purifiée | Aqua | Qsp 100 |
| EDTA tétrasodique | Tetrasodium EDTA | 0,05 |

| Phase B | | |
|---|---|---|
| N-Hance^{™} HP40S guar | Hydroxypropyl Guar | 0,10 |

| Phase C | | |
|---|---|---|
| Lubrajel^{™} DV Free hydrogel | Glycerin (and) Glyceryl Acrylate/Acrylic Acid Copolymer | 6,00 |

| Phase D | | |
|---|---|---|
| Si-Tec^{™} GF 3096 silicone | Dimethicone (and) Dimethiconol | 12,00 |
| RapiThix^{™} A-60 polymer | Sodium Polyacrylate (and) Hydrogenated Polydecene (and) Trideceth-6 | 2,40 |

| Phase E | | |
|---|---|---|
| Optiphen^{™} Plus preservative | Phenoxyethanol (and) Caprylyl Glycol (and) Sorbic Acid | 1,50 |

| Phase F | | |
|---|---|---|
| Surfin* 96 | Alcohol Denat. | 3,50 |
| PF Cucumber & Aloe | Parfum/Fragrance | 0,50 |

| Phase G | | |
|---|---|---|
| Extrait selon l'exemple 1, origine Inde | Propanediol (and) *Camellia Sinensis* Leaf Extract | 1,00 |
| Achromaxyl^{™} ISR biofunctional | Water/Aqua (and) Glycerin (and) Hydrolyzed Brassica Napus Seedcake Extract | 3,00 |
| Xirona Carribean Blue | Mica (and) CI 77891 (Titanium Dioxide) (and) Silica (and) Tin Oxide | 1,00 |

Procédé de préparation :
1. A 25°C, homogénéiser la phase A dans le récipient principal ;
2. A 25°C, saupoudrer dans la phase B et bien mélanger jusqu'à homogénéité ;
3. A 25°C, ajouter la phase C et bien mélanger jusqu'à homogénéité ;
4. Prémélanger la phase D dans un bécher à part et ajouter dans le récipient principal à 25°C ;
5. A 25°C, ajouter la phase E dans le récipient principal et bien mélanger ;
6. Prémélanger la phase F et l'ajouter lentement. Bien mélanger jusqu'à homogénéité ;
7. Prémélanger la phase G dans un bécher à part et ajouter dans le récipient principal jusqu'à homogénéité ;
8. Arrêter à 25°C.

La composition se présente ainsi sous forme d'un gel crème avec des effets vert scintillant, avec un pH compris entre 5,30 et 5,80 et une viscosité (D0) de 70000 - 100000 cps (Brookfield RVT/Spindle C/5 RPM/1 minute/25°C).

### Exemple 6 : Formule d'un Sérum

**[Tableau 14]**

| Ingrédients (Nom de marque) | INCI | % w/w |
|---|---|---|
| Phase A | | |
| Eau déminéralisée | Aqua | 87,40 |
| Sodium Hyaluronate | Sodium Hyaluronate | 0,20 |
| RapiThix^{™} A-60 polymer | Sodium Polyacrylate (and) Hydrogenated Polydecene (and) Trideceth-6 | 0,40 |
| Lubrajel^{™} DV hydrogel | Glycerin (and) Glyceryl Acrylate/Acrylic Acid Copolymer (and) Propylene Glycol | 6,00 |
| Lubrajel^{™} Oil hydrogel | Glycerin (and) Glyceryl Acrylate/Acrylic Acid Copolymer (and) Propylene Glycol (and) PVM/MA Copolymer | 1,00 |
| Wacker-Belsil* DM 100 | Dimethicone | 2,00 |
| Cyclopentasiloxane NF | Cyclopentasiloxane | 0,50 |
| Extrait selon l'exemple 1, origine Inde | Propanediol (and) *Camellia Sinensis* Leaf Extract | 1,00 |
| Optiphen^{™} preservative | Phenoxyethanol (and) Caprylyl Glycol | 1,50 |

Procédé de préparation :
1. Ajouter de l'eau dans le récipient principal et commencer le mélange avec une pale d'hélice hi-lo ;
2. Ajouter le reste des ingrédients, l'un après l'autre tout en mélangeant entre chaque addition.

La composition se présente ainsi sous forme d'un sérum lisse, semi-opaque, avec un pH compris entre 5,75 et 6,25 et une viscosité (D0) de 1,100 - 1,400 cps (Brookfield RVT/s

## Revendications

1. Extrait de feuilles de thé noir de l'espèce *Camellia sinensis* comprenant de 10 à 350 mg/kg de petits ARN d'une longueur d'au maximum 150 nucléotides, au maximum 150 mg/kg de catéchines, de préférence au maximum 120 mg/kg de catéchines et encore plus préférentiellement au maximum 30 mg/kg de catéchines, **caractérisé en ce que** l'extrait de thé noir (Camelia sinensis) est obtenu par le procédé d'obtention comprenant les étapes suivantes :
a) on met en présence les feuilles de thé préalablement broyées avec de l'eau ;
b) on ajoute de l'acide phytique ;
c) on ajuste le pH à une valeur comprise entre 10 et 11 ;
d) on maintient le mélange sous agitation pendant au moins une heure à une température comprise entre 40 et 80°C ;
e) on purifie le mélange obtenu en c) pour éliminer la matière végétale solide résiduelle et récolter le filtrat ;
f) on ajuste le pH à une valeur comprise entre 6 et 8 ;
g) on traite par du charbon actif en poudre ;
h) on filtre pour éliminer le charbon actif et récolter le filtrat ;
i) on traite le filtrat à l'aide de polyvinylpolypyrrolidones (PVPP) ;
j) on filtre pour éliminer les PVPP et récolter le filtrat ;
k) on contrôle et réajuste le pH du filtrat, si nécessaire, à une valeur comprise entre 6 et 6,5.
l) Optionnellement l'extrait peut être dilué dans un solvant physiologiquement acceptable puis son pH est ajusté à une valeur comprise entre 5,8 et 6,5 préférentiellement entre 6,0 et 6,5, pour obtenir un extrait dilué ayant un poids sec compris entre 4 à 20 g/kg.

2. Extrait de feuilles de thé noir selon la revendication 1, **caractérisé en ce qu'**il contient au maximum 0,001% de catéchines par rapport au poids sec de l'extrait, préférentiellement ne contient pas de catéchines.

3. Extrait de feuilles de thé noir selon l'une des revendications précédentes, **caractérisé en ce qu'**il comprend de 0,030 à 5 g/kg de composés phénoliques.

4. Extrait de feuilles de thé noir selon l'une des revendications précédentes, **caractérisé en ce qu'**il a un poids sec de 4 à 30 g/kg et qu'il comprend de 0,2 à 10 g/kg de sucres, de 0,010 à 3 g/kg d'acides aminés totaux, de 0,010 à 2 g/kg de théine et de 0,010 à 2 g/kg de théanine.

5. Extrait de feuilles de thé noir selon l'une des revendications précédentes, **caractérisé en ce qu'**il a été dilué dans un solvant physiologiquement acceptable, qu'il a un poids sec compris entre 4 et 20 g/kg et comprend en poids du poids total de l'extrait dilué, de 10 à 250 mg/kg d'ARN de petit poids moléculaire d'une longueur d'au maximum 150 nucléotides, de 0,2 à 5 g/kg de sucres, de 0,010 à 2 g/kg d'acides aminés, de 0,030 à 3 g/kg de composés phénoliques dont au maximum 120 mg/kg de catéchines, de 0,010 à 1 g/kg de théine, de 0,010 à 1 g/kg de théanine.

6. Procédé d'obtention d'un extrait de feuilles de thé noir *(Camellia sinensis),* tel que défini selon l'une quelconque des revendications 1 à 5, ledit procédé comprenant les étapes suivantes :
a) on met en présence les feuilles de thé préalablement broyées avec de l'eau ;
b) on ajoute de l'acide phytique ;
c) on ajuste le pH à une valeur comprise entre 10 et 11 ;
d) on maintient le mélange sous agitation pendant au moins une heure à une température comprise entre 40 et 80°C ;
e) on purifie le mélange obtenu en c) pour éliminer la matière végétale solide résiduelle et récolter le filtrat ;
f) on ajuste le pH à une valeur comprise entre 6 et 8 ;
g) on traite par du charbon actif en poudre ;
h) on filtre pour éliminer le charbon actif et récolter le filtrat ;
i) on traite le filtrat à l'aide de polyvinylpolypyrrolidones (PVPP) ;
j) on filtre pour éliminer les PVPP et récolter le filtrat ;
k) on contrôle et réajuste le pH du filtrat, si nécessaire, à une valeur comprise entre 6 et 6,5.
l) Optionnellement l'extrait peut être dilué dans un solvant physiologiquement acceptable puis son pH est ajusté à une valeur comprise entre 5,8 et 6,5 préférentiellement entre 6,0 et 6,5, pour obtenir un extrait dilué ayant un poids sec compris entre 4 à 20 g/kg.

7. Procédé selon la revendication 6, **caractérisé en ce que** lorsque l'extrait est dilué à l'étape I), le solvant physiologiquement acceptable est choisi parmi l'eau, le glycérol, l'éthanol, le propanediol, le butylène glycol, le dipropylène glycol, les diglycols éthoxylés ou propoxylés, les polyols cycliques ou tout mélange de ces solvants.

8. Procédé selon les revendications 6 ou 7, **caractérisé en ce qu'**à l'étape a) on met en présence les feuilles de thé noir avec de l'eau, dans un rapport matière végétale / eau compris entre 1 % et 20 %, préférentiellement entre 2 et 10 % et encore plus préférentiellement entre 2 et 5 %.

9. Procédé selon l'une des revendications 6 à 8, **caractérisé en ce qu'**à l'étape b) le traitement est réalisé avec de l'acide phytique à une concentration comprise entre 1 et 10 mM et préférentiellement entre 1 et 5 mM.

10. Composition comprenant, en tant qu'agent actif, une quantité efficace d'extrait de thé noir dilué selon la revendication 5, et un milieu physiologiquement acceptable.

11. Composition selon la revendication 10, dans laquelle ledit agent actif est présent à une concentration comprise entre 0,05 et 5 % en poids du poids total de la composition et préférentiellement entre 0,1 à 2,5 % en poids du poids total de la composition et un milieu physiologiquement acceptable.

12. Composition selon l'une des revendications 10 ou 11, **caractérisée en ce qu'**elle est formulée pour être appliquée topiquement sur la peau, les phanères et le cuir chevelu.

13. Composition selon l'une des revendications 10 à 12, **caractérisée en ce que** ledit agent actif est un extrait de thé noir de toute provenance à l'exclusion du thé noir provenant de l'Ile Maurice.

14. Utilisation non-thérapeutique de la composition de la revendication 13 pour le soin de la peau, du cuir chevelu et des phanères.

15. Utilisation non-thérapeutique de la composition de la revendication 13 pour obtenir les bénéfices cosmétiques choisis parmi : protéger la peau des agressions externes et de l'oxydation, lutter contre les signes du vieillissement cutané, augmenter la photoprotection, éclaircir la peau, ou encore améliorer l'hydratation de la peau.

## Patentansprüche

1. Extrakt aus schwarzen Teeblättern der Art *Camellia sinensis,* umfassend zu 10 bis 350 mg/kg kleine RNAs mit einer Länge von höchstens 150 Nukleotiden, höchstens zu 150 mg/kg Catechine, vorzugsweise höchstens zu 120 mg/kg Catechine und noch mehr bevorzugt höchstens zu 30 mg/kg Catechine, **dadurch gekennzeichnet, dass** der Schwarztee-Extrakt (Camellia sinensis) durch einen Gewinnungsvorgang gewonnen wird, umfassend die folgenden Schritte:
a) Inkontaktbringen der zuvor zerkleinerten Teeblätter mit Wasser;
b) Zugeben von Phytinsäure;
c) Anpassen des pH-Werts auf einen Wert zwischen 10 und 11;
d) Beibehalten eines Rührens der Mischung für mindestens eine Stunde bei einer Temperatur zwischen 40 und 80 °C;
e) Reinigen der in c) gewonnenen Mischung zum Entfernen des restlichen festen Pflanzenstoffs und Sammeln des Filtrats;
f) Anpassen des pH-Werts auf einen Wert zwischen 6 und 8;
g) Behandeln mit pulverisierter Aktivkohle;
h) Filtern zum Entfernen der Aktivkohle und Sammeln des Filtrats;
i) Behandeln des Filtrats mittels Polyvinylpolypyrrolidonen (PVPP);
j) Filtern zum Entfernen der PVPP und Sammeln des Filtrats;
k) Überprüfen des pH-Werts des Filtrats und neu Anpassen dessen, falls notwendig, auf einen Wert zwischen 6 und 6,5.
l) optional kann der Extrakt in einem physiologisch verträglichen Lösungsmittel verdünnt werden, wobei anschließend sein pH-Wert auf einen Wert zwischen 5,8 und 6,5, vorzugsweise zwischen 6,0 und 6,5, angepasst wird, um einen verdünnten Extrakt zu gewinnen, der ein Trockengewicht zwischen 4 und 20 g/kg aufweist.

2. Extrakt aus schwarzen Teeblättern nach Anspruch 1, **dadurch gekennzeichnet, dass** er höchstens zu 0,001 % Catechine, bezogen auf das Trockengewicht des Extrakts enthält, wobei er vorzugsweise keine Catechine enthält.

3. Extrakt aus schwarzen Teeblättern nach einem der vorstehenden Ansprüche, **dadurch gekennzeichnet, dass** er zu 0,030 bis 5 g/kg phenolische Verbindungen umfasst.

4. Extrakt aus schwarzen Teeblättern nach einem der vorstehenden Ansprüche, **dadurch gekennzeichnet, dass** er ein Trockengewicht von 4 bis 30 g/kg aufweist und zu 0,2 bis 10 g/kg Zucker, zu 0,010 bis 3 g/kg Gesamtaminosäuren, zu 0,010 bis 2 g/kg Thein und zu 0,010 bis 2 g/kg Theanin umfasst.

5. Extrakt aus schwarzen Teeblättern nach einem der vorstehenden Ansprüche, **dadurch gekennzeichnet, dass** er in einem physiologisch verträglichen Lösungsmittel verdünnt wurde, er ein Trockengewicht zwischen 4 und 20 g/kg aufweist und, bezogen auf das Gesamtgewicht des verdünnten Extrakts, zu 10 bis 250 mg/kg niedermolekulare RNA mit einer Länge von höchstens 150 Nukleotiden, zu 0,2 bis 5 g/kg Zucker, zu 0,010 bis 2 g/kg Aminosäuren, zu 0,030 bis 3 g/kg phenolische Verbindungen, darunter höchstens 120 mg/kg Catechine, zu 0,010 bis 1 g/kg Thein, zu 0,010 bis 1 g/kg Theanin, umfasst.

6. Verfahren zum Gewinnen eines Extrakts aus schwarzen Teeblättern *(Camellia sinensis),* wie in einem der Ansprüche 1 bis 5 definiert, das Verfahren umfassend die folgenden Schritte:
a) Inkontaktbringen der zuvor zerkleinerten Teeblätter mit Wasser;
b) Zugeben von Phytinsäure;
c) Anpassen des pH-Werts auf einen Wert zwischen 10 und 11;
d) Beibehalten eines Rührens der Mischung für mindestens eine Stunde bei einer Temperatur zwischen 40 und 80 °C;
e) Reinigen der in c) gewonnenen Mischung zum Entfernen des restlichen festen Pflanzenstoffs und Sammeln des Filtrats;
f) Anpassen des pH-Werts auf einen Wert zwischen 6 und 8;
g) Behandeln mit pulverisierter Aktivkohle;
h) Filtern zum Entfernen der Aktivkohle und Sammeln des Filtrats;
i) Behandeln des Filtrats mittels Polyvinylpolypyrrolidonen (PVPP);
j) Filtern zum Entfernen der PVPP und Sammeln des Filtrats;
k) Überprüfen des pH-Werts des Filtrats und neu Anpassen dessen, falls notwendig, auf einen Wert zwischen 6 und 6,5.
l) optional kann der Extrakt in einem physiologisch verträglichen Lösungsmittel verdünnt, anschließend sein pH-Wert auf einen Wert zwischen 5,8 und 6,5, vorzugsweise zwischen 6,0 und 6,5, angepasst werden, um einen verdünnten Extrakt zu gewinnen, der ein Trockengewicht zwischen 4 und 20 g/kg aufweist.

7. Verfahren nach Anspruch 6, **dadurch gekennzeichnet, dass,** wenn der Extrakt in Schritt l) verdünnt wird, das physiologisch verträgliche Lösungsmittel aus Wasser, Glycerin, Ethanol, Propandiol, Butylenglycol, Dipropylenglycol, ethoxylierten oder propoxylierten Diglycolen, cyclischen Polyolen oder einer beliebigen Mischung dieser Lösungsmittel ausgewählt wird.

8. Verfahren nach Anspruch 6 oder 7, **dadurch gekennzeichnet, dass** in Schritt a) die schwarzen Teeblätter mit Wasser in einem Verhältnis von Pflanzenstoff / Wasser zwischen 1 % und 20 %, vorzugsweise zwischen 2 und 10 % und noch mehr bevorzugt zwischen 2 und 5 % in Kontakt gebracht werden.

9. Verfahren nach einem der Ansprüche 6 bis 8, **dadurch gekennzeichnet, dass** in Schritt b) die Behandlung mit Phytinsäure in einer Konzentration zwischen 1 und 10 mM und vorzugsweise zwischen 1 und 5 mM durchgeführt wird.

10. Zusammensetzung, umfassend als Wirkstoff eine wirksame Menge des verdünnten Schwarztee-Extrakts nach Anspruch 5 und ein physiologisch verträgliches Medium.

11. Zusammensetzung nach Anspruch 10, wobei der Wirkstoff in einer Konzentration zwischen 0,05 und 5 Gew.-% des Gesamtgewichts der Zusammensetzung und vorzugsweise zwischen 0,1 bis 2,5 Gew.-% des Gesamtgewichts der Zusammensetzung und einem physiologisch verträglichen Medium vorhanden ist.

12. Zusammensetzung nach einem der Ansprüche 10 oder 11, **dadurch gekennzeichnet, dass** sie zum topischen Anwenden auf der Haut, den Hautanhangsgebilden und der Kopfhaut formuliert ist.

13. Zusammensetzung nach einem der Ansprüche 10 bis 12, **dadurch gekennzeichnet, dass** der Wirkstoff ein Extrakt aus schwarzem Tee beliebiger Herkunft ist, mit Ausnahme von schwarzem Tee, der aus Mauritius stammt.

14. Nichttherapeutische Verwendung der Zusammensetzung nach Anspruch 13 für die Pflege der Haut, der Kopfhaut und der Hautanhangsgebilde.

15. Nichttherapeutische Verwendung der Zusammensetzung nach Anspruch 13 zum Gewinnen der kosmetischen Vorteile, die ausgewählt sind aus: Schützen der Haut vor äußeren Einflüssen und Oxidation, Bekämpfen der Zeichen der Hautalterung, Erhöhen des Lichtschutzes, Aufhellen der Haut oder weiter Verbessern der Hautfeuchtigkeit.

## Claims

1. Extract of black tea leaves of the species *Camellia sinensis* comprising from 10 to 350 mg/kg of small RNA having a maximum length of 150 nucleotides, a maximum of 150 mg/kg of catechins, preferably a maximum of 120 mg/kg of catechins and even more preferably a maximum of 30 mg/kg of catechins, **characterized in that** the extract of black tea (Camellia sinensis) is obtained by the production method comprising the following steps:
a) placing the previously ground tea leaves in water;
b) adding phytic acid;
c) adjusting the pH to a value of between 10 and 11;
d) keeping the mixture agitated for at least one hour at a temperature of between 40 and 80°C;
e) purifying the mixture obtained in step c) in order to remove the residual solid plant matter and collect the filtrate;
f) adjusting the pH to a value of between 6 and 8;
g) treating with powdered activated carbon;
h) filtering in order to remove the activated carbon and collect the filtrate;
i) treating the filtrate with polyvinylpolypyrrolidones (PVPP);
j) filtering in order to remove the PVPP and collect the filtrate;
k) monitoring and readjusting the pH of the filtrate, if necessary, to a value of between 6 and 6.5.
l) Optionally, the extract can be diluted in a physiologically acceptable solvent then its pH is adjusted to a value of between 5.8 and 6.5, preferably between 6.0 and 6.5, to obtain a diluted extract with a dry weight of between 4 and 20 g/kg.

2. Black tea leaf extract according to claim 1, **characterized in that** it contains a maximum of 0.001% catechins relative to the dry weight of the extract, preferably it does not contain catechins.

3. Black tea leaf extract according to one of the preceding claims, **characterized in that** it comprises from 0.030 to 5 g/kg of phenolic compounds.

4. Black tea leaf extract according to one of the preceding claims, **characterized in that** it has a dry weight of 4 to 30 g/kg and comprises 0.2 to 10 g/kg of sugars, 0.010 to 3 g/kg of total amino acids, 0.010 to 2 g/kg of theine and 0.010 to 2 g/kg of theanine.

5. Black tea leaf extract according to one of the preceding claims, **characterized in that** it has been diluted in a physiologically acceptable solvent, that it has a dry weight of between 4 and 20 g/kg and comprises, by weight of the total weight of the diluted extract, from 10 to 250 mg/kg of low molecular weight RNA having a maximum length of 150 nucleotides, from 0.2 to 5 g/kg of sugars, from 0.010 to 2 g/kg of amino acids, from 0.030 to 3 g/kg of phenolic compounds which include a maximum of 120 mg/kg of catechins, from 0.010 to 1 g/kg of theine, from 0.010 to 1 g/kg of theanine.

6. Method for producing an extract of the leaves of black tea *(Camellia sinensis),* as defined according to any one of claims 1 to 5, said method comprising the following steps:
a) placing the previously ground tea leaves in water;
b) adding phytic acid;
c) adjusting the pH to a value of between 10 and 11;
d) keeping the mixture agitated for at least one hour at a temperature of between 40 and 80°C;
e) purifying the mixture obtained in step c) in order to remove the residual solid plant matter and collect the filtrate;
f) adjusting the pH to a value of between 6 and 8;
g) treating with powdered activated carbon;
h) filtering in order to remove the activated carbon and collect the filtrate;
i) treating the filtrate with polyvinylpolypyrrolidones (PVPP);
j) filtering in order to remove the PVPP and collect the filtrate;
k) monitoring and readjusting the pH of the filtrate, if necessary, to a value of between 6 and 6.5.
l) Optionally, the extract can be diluted in a physiologically acceptable solvent then its pH is adjusted to a value of between 5.8 and 6.5, preferably between 6.0 and 6.5, to obtain a diluted extract with a dry weight of between 4 and 20 g/kg.

7. Method according to claim 6, **characterized in that** when the extract is diluted in step I), the physiologically acceptable solvent is selected from water, glycerol, ethanol, propanediol, butylene glycol, dipropylene glycol, ethoxylated or propoxylated diglycols, cyclic polyols or any mixture of these solvents.

8. Method according to claims 6 or 7, **characterized in that** in step a) the black tea leaves are placed in water, in a plant matter/water ratio of between 1% and 20%, preferably between 2 and 10% and even more preferably between 2 and 5%.

9. Method according to one of claims 6 to 8, **characterized in that** in step b) the treatment is carried out with phytic acid at a concentration of between 1 and 10 mM and preferably between 1 and 5 mM.

10. Composition comprising, as an active agent, an effective amount of black tea extract diluted according to claim 5, and a physiologically acceptable medium.

11. Composition according to claim 10, wherein said active agent is present at a concentration of between 0.05 and 5% by weight of the total weight of the composition and preferably between 0.1 and 2.5% by weight of the total weight of the composition and a physiologically acceptable medium.

12. Composition according to one of claims 10 or 11, **characterized in that** it is formulated to be applied topically to the skin, skin appendages and the scalp.

13. Composition according to one of claims 10 to 12, **characterized in that** said active agent is an extract of black tea from any source with the exception of black tea from Mauritius.

14. Non-therapeutic use of the composition of claim 13 for caring for the skin, the scalp and skin appendages.

15. Non-therapeutic use of the composition of claim 13 for obtaining cosmetic benefits selected from: protecting the skin from external aggression and oxidation, combating the signs of skin aging, increasing photoprotection, lightening the skin, or improving skin hydration.
